# DEMANDE DE BREVET EUROPEEN

(11) **EP 1 847 254 A1**
(43) Date de publication de la demande: **24.10.2007**
(21) Numéro de dépôt: 07300954.0
(22) Date de dépôt: 16.04.2007
(51) Int. Cl.: A61K 8/92, A61Q 1/10, A45D 40/26, A46B 9/02

(54) **Kit de maquillage et/ou de soin**

(30) Priorité: 21.04.2006 FR 0651410
(71) Demandeur: L'ORÉAL, 75008 Paris (FR)
(72) Inventeur: Bodelin, Sophie, 92170, VANVES (FR)
(74) Mandataire: Le Coupanec, Pascale A.M.P.

(57) **Abrégé**

La présente invention concerne un kit de maquillage et/ou de soin des cils caractérisé en ce qu'il comprend ;
- au moins une composition (i) présentant une teneur totale en cire(s) et en polymère(s) hydrophile(s) inférieure ou égale à 26 % en poids par rapport au poids total de la composition,
- au moins une composition (ii) présentant une teneur total en cire(s) et en polymère(s) filmogène(s) hydrophile(s) supérieure à 26 % en poids par rapport au poids total de la composition,
au moins l'une desdites compositions (i) et (ii) comprenant une phase aqueuse continue, et en ce qu'il comprend un moyen d'application sur la frange de cils, comportant une pluralité d'éléments d'application (6 ; 60) disposés sur un support (5) sous forme d'au moins une rangée dont la longueur est telle que les éléments d'application (6 ; 60) puissent au mieux contacter simultanément au plus un quart des cils de la frange.

## Description

La présente invention concerne un kit de maquillage et/ou de soin des cils comprenant au moins une composition cosmétique de maquillage et/ou de soin des cils et au moins une composition cosmétique de maquillage et/ou de soin des cils chargeante comprenant un moyen d'application permettant une application de ladite composition cosmétique chargeante sur seulement une portion de la frange des cils, et en particulier sur au plus le tiers extérieur de la frange des cils.

Il existe un besoin d'obtenir de nouveaux effets de maquillage sur les cils et notamment de déposer du mascara de façon plus prononcée sur seulement une portion de la frange des cils.

A l'aide d'un applicateur de type brosse ou peigne classique, il peut s'avérer délicat de faire un dépôt de matière précis sur seulement une portion des cils, tout en évitant la formation d'amas.

Il peut en effet être intéressant, pour obtenir des effets de maquillage particuliers, de jouer sur des dépôts de matière contrastés sur des zones disjointes de la frange des cils.

Les inventeurs ont constaté qu'un maquillage plus important d'une partie des cils, en particulier un dépôt important de matière sur au plus un tiers de la frange des cils, et plus particulièrement, sur la partie extérieure de la frange des cils, procure un résultat maquillage particulier qui ouvre le regard et agrandit l'oeil, en modifiant optiquement la perception de la forme de l'oeil. Un tel maquillage comportant un dépôt de matière plus important sur au plus un tiers de la frange, sur l'extérieur de la frange, donne une impression de forme d'oeil en amande et/ou d'oeil étiré, dont le coin extérieur est remonté (effet « lifting » du regard).

L'invention vise donc un moyen pour permettre d'atteindre facilement cet objectif de maquillage particulier.

La présente invention a donc pour objet, selon un de ses aspects un kit de maquillage et/ou de soin des cils caractérisé en ce qu'il comprend :
- au moins une composition (i) présentant une teneur totale en cire(s) et en polymère(s) hydrophile(s) inférieure ou égale à 26 % en poids par rapport au poids total de la composition,
- au moins une la composition (ii) présentant une teneur totale en cire(s) et en polymère(s) filmogène(s) hydrophile(s) supérieure à 26 % en poids par rapport au poids total de la composition,
au moins l'une desdites compositions (i) et (ii) comprenant une phase aqueuse continue, et en ce qu'il comprend un moyen d'application sur la frange de cils, comportant une pluralité d'éléments d'application (6 ; 60) disposés sur un support (5) sous forme d'au moins une rangée dont la longueur est telle que les éléments d'application (6 ; 60) puissent au mieux contacter simultanément au plus un quart des cils de la frange.

Les inventeurs ont ainsi observé que l'application sur les cils de la composition (i) et de la composition (ii), dont l'une au moins est appliquée à l'aide de ce moyen d'application particulier, et plus préférentiellement la composition (ii), permet d'effectuer aisément un dépôt plus volumateur ou chargeant sur seulement une partie de la frange des cils, par exemple sur au plus le tiers extérieur de la frange des cils.

En particulier, la composition (i) permet d'obtenir un dépôt lisse et homogène, facile à appliquer, qui gaine, sépare et/ou allonge les cils. La composition (i) permet d'obtenir un maquillage peu chargeant, c'est à dire qu'il n'épaissit pas les cils : on obtient ainsi un maquillage naturel. On peut ainsi effectuer aisément sur ce premier film de maquillage un dépôt plus volumateur ou chargeant sur seulement une partie de la frange des cils, par exemple sur au plus le tiers extérieur de la frange des cils par application de la composition (ii), qui de par sa teneur totale en cires et polymère(s) hydrophile(s) permet un dépôt de matière plus important.

Ainsi, ce kit permet d'accentuer le contraste entre le maquillage procuré par le dépôt de la composition (i) et celui procuré par le dépôt de la composition (ii), tout en évitant la formation de "paquets" jugés inesthétiques.

Selon un deuxième aspect, la présente invention concerne un procédé de maquillage et/ou de soin non thérapeutique des cils, caractérisé en ce qu'il comprend :
- au moins une étape d'application sur les cils d'au moins une couche d'une composition (i) présentant une teneur totale en cire(s) et en polymère(s) hydrophile(s) inférieure ou égale à 26 % en poids par rapport au poids total de la composition, et
- au moins une étape d'application d'au moins une couche d'une composition (ii), présentant une teneur totale en cire(s) et en polymère(s) filmogène(s) hydrophile(s) supérieure à 26 % en poids par rapport au poids total de la composition, au moins l'une desdites compositions (i) et (ii) comprenant une phase aqueuse continue, et l'étape d'application de la composition (ii) étant effectuée à l'aide du moyen d'application comportant une pluralité d'éléments d'application (6 ; 60) disposés sur un support (5) sous forme d'au moins une rangée dont la longueur est telle que les éléments d'application (6 ; 60) puissent au mieux contacter simultanément au plus un quart des cils de la frange.

Selon un troisième aspect, la présente invention a pour objet un support maquillé, tel que des faux cils, comprenant un maquillage susceptible d'être obtenu selon le procédé tel que défini précédemment.

Les kits de maquillage conformes à l'invention comprennent un milieu physiologiquement acceptable, notamment cosmétiquement acceptable, c'est-à-dire un milieu compatible en particulier avec les cils et la zone oculaire.

L'utilisatrice peut obtenir l'effet maquillage par dépôt contrasté de matière par au moins deux gestes d'application.

L'ordre d'application de la composition (i) et de la composition (ii) peut varier. On préfère toutefois appliquer la composition (i) préalablement à la composition (ii).

Suivant l'ordre d'application, l'une ou l'autre des compositions cosmétiques peut être qualifiée de "basecoat" ou de "topcoat".

Par "cosmétiquement acceptable", on entend, dans le cadre de la présente invention, un composé dont l'utilisation est compatible avec une application sur les cils.

Par "frange" au sens de la présente invention, on désigne les cils de la paupière supérieure ou ceux de la paupière inférieure, du coin intérieur au coin extérieur de la paupière, ou encore les sourcils de l'arcade sourcilière de l'un ou l'autre des yeux de l'utilisateur.

L'expression "comportant un" doit être considérée comme étant synonyme de l'expression "comportant au moins un", et l'expression "compris entre" doit être considérée comme incluant les bornes, sauf si le contraire est spécifié.

Toutes les teneurs en composants sont exprimées en matière sèche.

Les termes "moyen d'application" ou "applicateur" sont employés indifféremment dans la suite de la description.

Par composition à phase continue aqueuse, on entend que la composition présente une conductivité, mesurée à 25 °C, supérieure ou égale à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

Selon un mode de réalisation préféré les deux compositions (i) et (ii) comprennent une phase continue aqueuse.

Les compositions (i) conformes à l'invention, présentent une teneur totale en cire(s) et en polymère(s) hydrophile(s) inférieure ou égale à 26 % en poids par rapport au poids total de la composition, de préférence inférieure ou égale à 24 % en poids et mieux inférieure ou égale à 23,5% en poids.

Elles comprennent de préférence une teneur totale en cire(s) et en polymère(s) hydrophile(s) supérieure ou égale à 10 % en poids, de préférence supérieure ou égale à 15% en poids par rapport au poids total de la composition (i).

Les compositions (ii) conformes à l'invention, de préférence à phase continue aqueuse, présentent selon un mode de réalisation privilégié une teneur totale cire(s) et en polymère(s) hydrophile(s) supérieure à 26 % en poids par rapport au poids total de la composition, de préférence supérieure ou égale à 27 % en poids et mieux supérieure ou égale à 28% en poids.

Elles peuvent présenter une teneur totale en cire(s) et en polymère(s) hydrophile(s) allant jusqu'à 50 % en poids, par rapport au poids total de la composition (ii).

Selon un mode de réalisation particulier de l'invention, le kit est tel que la différence entre la teneur totale en cire(s) et en polymère(s) hydrophile(s) de la composition (ii) et la teneur totale en cire(s) et en polymère(s) hydrophile(s) de la composition (i) est supérieure ou égale à 2 %, voire 3 %, et mieux à 4 % en valeur absolue.

### CIRES

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

La cire peut être présente en une teneur allant de 0,1 à 50 % en poids par rapport au poids total de chaque composition (i) et (ii), mieux de 1 à 40 % et encore mieux de 5 à 30% en poids.

Selon une variante privilégiée de l'invention, la teneur en cire(s) dans la composition (i) varie de 1 à 40 %, de préférence de 5 à 30 %, et mieux de 8 à 25 % en poids, par rapport au poids total de la composition.

Selon une autre variante privilégiée de l'invention, la teneur en cire(s) dans la composition (ii) varie de 5 à 40 %, de préférence de 15 à 30 %, et mieux de 18 à 25 % en poids, par rapport au poids total de la composition.

Aussi, de façon tout particulièrement préférée, le kit comprend une composition (i) présentant une teneur en cire(s) comprise ente 8 et 25 % en poids par rapport à son poids total et une composition (ii) présentant une teneur en cire(s) comprise entre 18 et 25 % en poids, par rapport à son poids total.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.

On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A- 2792190.

Selon un mode de réalisation particulier, les compositions (i) ou (ii) conformes à l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.

Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.
Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.
Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.
La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.
La dureté est mesurée selon le protocole décrit précédemment.
Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II) : 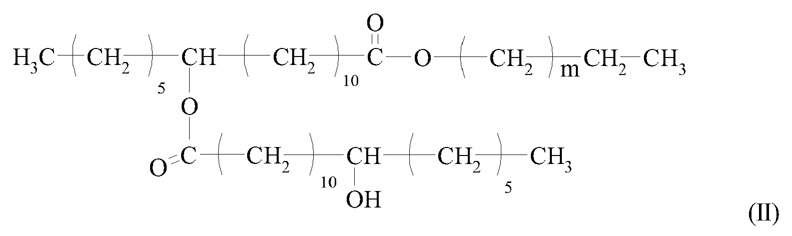
dans laquelle m est un entier allant de 18 à 38, ou un mélange de composés de formule (II).

Une telle cire est notamment vendue sous les dénominations "Kester Wax K 82 P®" et "Kester Wax K 80 P®" par la société KOSTER KEUNEN.

Les cires citées ci-dessus présentent généralement un point de fusion commençante inférieur à 45 °C.

On peut également utiliser la cire microcristalline commercialisée sous la référence SP18 par la société STRAHL and PITSCH qui présente une dureté d'environ 0,46 MPa et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

### POLYMERE HYDROPHILE

Le polymère hydrophile peut être choisi en particulier parmi les polymères filmogènes hydrophiles, les gélifiants hydrophiles et leurs mélanges, certains polymères filmogènes hydrophiles pouvant également jouer le rôle de gélifiant.

Dans la composition (i), la teneur totale en matières sèches de polymère(s) hydrophile(s) peut être comprise entre 0,5 et 15 %, de préférence entre 1 et 10 % en poids par rapport au poids total de la composition.

Dans la composition (ii), la teneur totale en matières sèches de polymère(s) hydrophile(s) peut être comprise entre 1 et 10 % en poids par rapport au poids total de la composition.

On préfère tout particulièrement dans le cadre de la présente invention, les kits présentant une composition (i) présentant une teneur totale en polymère(s) hydrophile(s) comprise entre 1 et 10 % en poids par rapport au poids total de la composition et une composition (ii) présentant une teneur totale en polymère(s) hydrophile(s) comprise entre 1 et 10 % en poids par rapport au poids total de la composition.

### I) Polymère filmogène

Dans la présente invention, on entend par "polymère filmogène", un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

De façon générale, la teneur en matières séches en polymère filmogène dans chacune des compositions (i) ou (ii) peut varier de 0,1 à 40 %, de préférence de 0,5 à 30 % et mieux de 1 à 20 % en poids, par rapport au poids total de la composition, Le polymère filmogène hydrophile peut être un polymère hydrosoluble ou se présenter en dispersion dans un milieu aqueux.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate, et leurs mélanges.
   Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.
   Comme dispersion aqueuse de polymère filmogène, on peut utiliser les dispersions acryliques vendues sous les dénominations Neocryl XK-90^{®}, Neocryl A-1070^{®}, Neocryl A-1090^{®}, Neocryl BT-62^{®}, Neocryl A-1079^{®} et Neocryl A-523^{®} par la société AVECIA-NEORESINS, Dow Latex 432^{®} par la société DOW CHEMICAL, Daitosol 5000 AD^{®} ou Daitosol 5000 SJ^{®} par la société DAITO KASEY KOGYO; Syntran 5760^{®} par la société Interpolymer Allianz Opt^{®} par la société Rohm and Haas ou encore les dispersions aqueuses de polyuréthane vendues sous les dénominations Neorez R-981^{®} et Neorez R-974^{®} par la société AVECIA-NEORESINS, les Avalure UR-405^{®}, Avalure UR-410^{®}, Avalure UR-425^{®}, Avalure UR-450^{®}, Sancure 875^{®}, Avalure UR-445^{®} et Sancure 2060^{®} par la société NOVEON, Impranil 85^{®} par la société BAYER, Aquamere H-1511^{®} par la société HYDROMER; les sulfopolyesters vendus sous le nom de marque Eastman AQ^{®} par la société EASTMAN CHEMICAL PRODUCTS, les dispersions vinyliques comme le Mexomère PAM^{®}, les dispersions aqueuses de polyvinyl acétate comme le "Vinybran^{®}" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur^{®}" par la société AIR PRODUCTS ou "Duromer^{®}" de NATIONAL STARCH, les dispersions type core/shell : par exemple celles commercialisées par la société ATOFINA sous la référence Kynar (core : fluoré- shell : acrylique) ou encore ceux décrits dans le document US 5 188 899 (core ; silice - shell : silicone) et leurs mélanges.

Selon un mode particulier de réalisation, la composition (i) ou (ii) conforme à l'invention comprend à titre de polymères filmogènes hydrophiles au moins, l'association d'un polymère cationique et d'un polymère anionique.

Le polymère cationique peut être choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylaminoalkyle quaternisés ou non, les polymères quaternaires de vinylpyrrolidone et de vinylimidazole, les polyamidoamines et leurs mélanges.

De préférence, le polymère cationique est une hydroxyalkyl(C₁-C₄)cellulose comportant des groupements ammonium quaternaires.

Le polymère anionique est avantageusement choisi parmi :
- A) les homo- ou copolymères d'acide acrylique ou méthacrylique ou leurs sels, les copolymères d'acide acrylique et d'acrylamide et leurs sels, les sels de sodium d'acides polyhydroxycarboxyliques tels que les copolymères d'acide acrylique et d'acrylamide vendus sous la forme de leur sel de sodium sous les dénominations RETEN^{®} par la société HERCULES, le polyméthacrylate de sodium vendu sous la dénomination DARVAN N°7^{®} par la société VANDERBILT, les sels de sodium d'acides polyhydroxycarboxyliques vendus sous la dénomination HYDAGEN F^{®} par la société HENKEL,
B) les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol; les copolymères de ce type comportant dans leur chaîne un motif acrylamide éventuellement N-alkylé et/ou hydroxyalkylé, les copolymères d'acide acrylique et de méthacrylate d'alkyle en C1-C4 et les terpolymères de vinylpyrrolidone, d'acide acrylique et de méthacrylate d'alkyle en C1-C20 ;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ;
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées ;
E) les polyacrylamides comportant des groupements carboxylates,
F) l'acide désoxyribonucléique ;
G) les copolymères d'au moins un diacide carboxylique, d'au moins un diol et d'au moins un monomère aromatique bifonctionnel portant un groupement -SO3M avec M représentant un atome d'hydrogène, un ion ammonium NH4+ ou un ion métallique ;
- et leurs mélanges.

Les polymères anioniques les plus particulièrement préférés sont choisis parmi les polymères anioniques non réticulés comme les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP et les homopolymères d'acide acrylique ou méthacrylique commercialisés par exemple sous la dénomination VERSICOL E 5 OU le polyméthacrylate de sodium vendu sous la dénomination DARVAN 7 par la société VANDERBILT, et leurs mélanges.

De préférence, le polymère anionique est un polyméthacrylate de sodium.

Chacune des compositions (i) ou (ii) conformes à l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### II) Gélifiant hydrophile

Les gélifiants hydrophiles utilisables dans les compositions selon l'invention peuvent être choisi parmi :
- les homo- ou copolymères d'acides acrylique ou méthacrylique ou leurs sels et leurs esters et en particulier les produits vendus sous les dénominations VERSICOL F^{®} ou VERSICOL K^{®} par la société ALLIED COLLOID, UTRAHOLD 8^{®} par la société CIBA-GEIGY, les acides polyacryliques de type SYNTHALEN K,
- les copolymères d'acide acrylique et d'acrylamide vendus sous la forme de leur sel de sodium sous les dénominations RETEN^{®} par la société HERCULES, les sels de sodium d'acides polyhydroxycarboxyliques vendus sous la dénomination HYDAGEN F^{®} par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL^{®} ou SIMULGEL^{®} commercialisés par la société SEPPIC, et
- les copolymères AMPS/méthacrylates d'alkyle polyoxyéthylénés (réticulés ou non), et leurs mélanges.
   Certains polymères filmogènes hydrosolubles cités plus haut peuvent également jouer le rôle de gélifiant hydrosoluble ;
- les polyuréthanes associatifs tels que le polymère C16-OE120-C16 de la société SERVO DELDEN (commercialisé sous le nom SER AD FX1100, molécule à fonction uréthanne et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vensus sous forme pure) ou le DW 1206B de chez RHOM & HAAS à chaîne alkyle en C20 et à liaison uréthane, vendu à 20 % en matière active dans l'eau. On peut aussi utiliser des solutions ou dispersions de ces polyuréthanes associatifs notamment dans l'eau ou en milieu hydroalcoolique. A titre d'exemple, de tels polymères on peut citer, le SER AD fx1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,
- et leurs mélanges.

Les gélifiants hydrophiles peuvent être présents dans chacune des compositions (i) et (ii) selon l'invention en une teneur allant de 0,05 à 40% en poids par rapport au poids total de la composition, de préférence de 0,5 à 20% et mieux de 1 à 15% en poids.

### Polymère filmogène additionnel

Chacune des compositions (i) ou (ii) conformes à l'invention, peut comprendre en outre au moins un polymère additionnel lipophile qui peut être soluble dans une phase solvant non aqueuse ou en dispersion dans une phase solvant non aqueuse.

Selon un mode de réalisation particulier, la composition comprend comme polymère filmogène additionnel un terpolymère acrylique particulier. Ce terpolymère présente l'avantage de procurer une bonne accroche sur les cils lors de l'application, notamment un dépôt de matière au bout des cils de manière à obtenir un effet allongeant et permet un dépôt lisse et homogène de matière lorsqu'il est appliqué sur les cils.

Selon un mode de réalisation particulier, le kit comprend une composition (i) contenant ce terpolymère, et une composition (ii) dépourvue d'un tel terpolymère.

Un tel type polymère et un mascara le comprenant sont plus particulièrement décrits dans le document EP 1647268.

L'enseignement de ce document est incorporé par référence à la présente demande.

Le terpolymère particulier est toutefois décrit ci-après en détail.

Le polymère terpolymère est un polymère résultant de la copolymérisation :
- d'au moins un monomère A choisi parmi les esters issus de la réaction de l'acide (meth)acrylique avec un monoalcool comprenant de 2 à 20 atomes de carbone,
- d'au moins un monomère B choisi parmi les esters issus de la réaction d'acide méthacrylique avec un monoalcool comprenant de 1 à 10 atomes de carbone,
- d'au moins un monomère C choisi parmi les N-vinyl lactames.

### Monomère A

Le monomère A est choisi parmi les esters issus de la réaction de l'acide (méth)acrylique avec un monoalcool comprenant de 2 à 20 atomes de carbone.

Selon un mode de réalisation, le copolymère comprend au moins un monomère A résultant de la réaction de l'acide méthacrylique avec un monoalcool comprenant de 5 à 20 atomes de carbone, de préférence de 7 à 18 atomes de carbone et mieux de 10 à 18 atomes de carbone.

En particulier, le monoalcool peut être choisi parmi : le 3-heptanol, le 1-octanol, le 2 octanol, l'alcool isooctylique, le 2-éthyl-1-hexanol, le 1-decanol, le 1-dodecanol, le 1-tridecanol, le 1-tetradecanol, le 1-octadecanol et leurs mélanges.

Le polymère selon l'invention peut aussi comprendre au moins un monomère A résultant de la réaction de l'acide acrylique avec un monoalcool comprenant de 2 à 15 atomes de carbone, de préférence de 4 à 14 atomes de carbone .

En particulier, le monoalcool en C2-C15 peut être choisi parmi : l'éthanol, le 1-butanol, le 2-butanol, le 1-pentanol, le 2-pentanol, le 3-pentanol, le 2-methyl-1-butanol, le 1-hexanol, le 2-hexanol, le 2-methyl-1-pentanol, le 3-methyl-1-pentanol, le 2-ethyl-1-butanol, le 3,5,5-trimethyl-1-hexanol, le 3-heptanol, le 1-octanol, le 2-octanol, l'alcool d'isooctyl, le 2-ethyl-1-hexanol, le 1-decanol, le 1-dodecanol, le 1-tridecanol, le 1-tetradecanol et leurs mélanges.

Avantageusement, le monomère A est choisi parmi le n-butyl acrylate, l'isooctyl acrylate, le lauryl methacrylate (issu de la réaction de l'acide méthacrylique et du 1-dodecanol) et leurs mélanges.

Avantageusement, le monomère A est présent en une proportion en nombre allant de 15 à 80%, mieux de 40 à 60% par rapport au nombre total de monomères du polymère.

### Monomère B

Le monomère B est choisi parmi les esters issus de la réaction d'acide méthacrylique avec un monoalcool comprenant de 1 à 10 atomes de carbone, de préférence de 1 à 6 et mieux de 1 à 4 atomes de carbone.

En particulier, le monoalcool peut être choisi parmi le méthanol, féthanol, le 1-propanol, le 2-propanol, le 1-butanol, le 2-butanol, le 1-pentanol, le 2-pentanol, le 3-pentanol et leurs mélanges.

De préférence, le monomère B est choisi parmi le méthylméthacrylate, le n-butyl méthacrylate et leurs mélanges.

Avantageusement, le monomère B est présent en une proportion en nombre allant de 20 à 70%, mieux de 25 à 50% par rapport au nombre total de monomères du polymère.

### Monomère C

Le monomère C est avantageusement choisi parmi les N-vinyl lactames (dérivés N-substitués de lactame) telles que celles décrites dans le document US 3 907 720, et en particulier parmi les N-vinyl lactames de formule suivante : 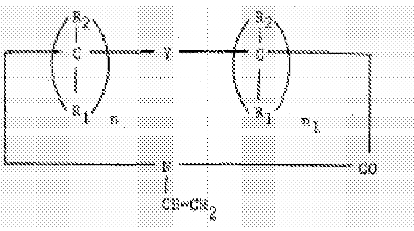 dans laquelle :
R1 et R2 représentent indépendamment un atome d'hydrogène, un groupement alkyl en C1-C5 tel que méthyle, éthyle, propyle, un groupement aryl ,
Y est choisi parmi O , S , -SO₂- , -N- , 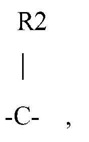 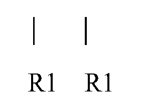 et n et n1 vont de 0 à 5, sous réserve que n et n1 ne soient pas simultanément égaux à 0.

De préférence, R1 et R2 représentent indépendamment un atome d'hydrogène, un groupement alkyl en C1-C5 tel que méthyle, éthyle, propyle et

Y représente

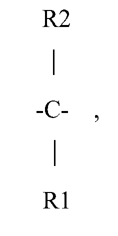

A titre de N-vinyl lactames utilisables comme monomères C, on peut citer la N-vinyl pyrrolidone, les dérivés N-vinyl substitués des lactames suivantes : la 3,3-diméthyl-1-pyrrolidone, la 4,4-diméthyl-2-pyrrolidone, la 3,4-diméthyl-2-pyrrolidone, la 3-éthyl-2-pyrrolidone, et la 3,5-diméthyl-2-pyrrolidone. De préférence, le monomère C est la N-vinyl pyrrolidone.

Avantageusement, le monomère C est présent en une proportion en nombre allant de 1 à 15%, mieux de 5 à 15% par rapport au nombre total de monomères du polymère.

Avantageusement, le polymère se présente en solution ou en dispersion dans un solvant organique qui est de préférence le "premier solvant organique" de la phase solvant organique de la composition (i) conforme à l'invention.

Le copolymère de la composition selon l'invention peut être préparé par les méthodes conventionnelles de polymérisation radicalaire, en particulier dans un solvant dans lequel les monomères sont solubles.

De tels copolymères et des compositions anti-microbiennes les contenant sont notamment décrits dans le document US 4 584 192.

Le polymère filmogène additionnel peut représenter de 0,01 à 20% en poids en matières sèche (ou matière active) par rapport au poids total de la composition (i) ou (ii), de préférence de 0,05 à 15% en poids, et mieux de 0,05 à 10% en poids.

### PHASE AQUEUSE

Au moins l'une des compositions (i) ou (ii) conformes à l'invention, de préférence chaque composition (i) et (ii), comprend une phase aqueuse continue comprenant de l'eau et/ou au moins un solvant hydrosoluble.

La phase aqueuse continue comprend de l'eau et/ou au moins un solvant hydrosoluble, qui peut former la phase continue de la composition.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions (i) ou (ii) conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄.

La phase aqueuse (eau et éventuellement le solvant miscible à l'eau) peut être présente dans chque composition (i) et (ii) en une teneur allant de 1 % à 95 % en poids, par rapport au poids total de la composition, de préférence allant de 3 % à 80 % en poids, et préférentiellement allant de 5 % à 60 % en poids.

### AUTRES COMPOSANTS CONTENUS DANS LES COMPOSITIONS (i) ET (ii)

### Système émulsionnant

Chacune des compositions (i) ou (ii) conformes à l'invention peut contenir des agents tensioactifs émulsionnants présents notamment en une proportion allant de 0,1 à 30 % en poids par rapport au poids total de la composition, mieux de 1 à 15 % et mieux de 2 à 10 % en poids.

Selon l'invention, on utilise généralement un émulsionnant choisi de manière appropriée pour l'obtention d'une émulsion cire dans eau ou huile-dans-eau. En particulier, on peut utiliser un émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces agents tensioactifs peuvent être choisis parmi des agents tensioactifs non ioniques, anioniques, cationiques, amphotériques ou encore des émulsionnants tensioactifs. On peut se reporter au document "Encyclopedia of Chemical Technology, KIRK-OTHMER", volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

Les tensioactifs utilisés préférentiellement dans la composition selon l'invention sont choisis parmi :
a) les agents tensioactifs non ioniques de HLB supérieur ou égal à 8 à 25 °C, utilisés seuls ou en mélange; on peut citer notamment :
   - les éthers oxyéthylénés et/ou oxypropylénés (pouvant comporter de 1 à 150 groupes oxyéthylénés et/ou oxypropylénés) de glycérol ;
   - les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination NEODOL 25-7^{®} par SHELL CHEMICALS,
   - les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P^{®} par la société ICI UNIQUEMA,
   - les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM^{®} par la société SEPPIC ; le stéarate de glycéryle polyéthoxylé à 30 groupes d'oxyde d'éthylène comme le produit TAGAT S^{®} vendu par la société GOLDSCHMIDT, l'oléate de glycéryle polyéthoxylé à 30 groupes d'oxyde d'éthylène comme le produit TAGAT O^{®} vendu par la société GOLDSCHMIDT, le cocoate de glycéryle polyéthoxylé à 30 groupes d'oxyde d'éthylène comme le produit VARIONIC LI 13^{®} vendu par la société SHEREX, l'isostéarate de glycéryle polyéthoxylé à 30 groupes d'oxyde d'éthylène comme le produit TAGAT L^{®} vendu par la société GOLDSCHMIDT et le laurate de glycéryle polyéthoxylé à 30 groupes d'oxyde d'éthylène comme le produit TAGAT I^{®} de la société GOLDSCHMIDT,
   - les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60^{®} par la société UNIQUEMA,
   - la diméthicone copolyol, telle que celle vendue sous la dénomination Q2-5220^{®} par la société DOW CORNING,
   - la diméthicone copolyol benzoate (FINSOLV SLB 101^{®} et 201^{®} de la société FINTEX),
   - les copolymères d'oxyde propylène et d'oxyde d'éthylène, également appelés polycondensats OE/OP,
   - et leurs mélanges.
   Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

   H-(O-CH₂-CH₂)ₐ-(O-CH(CH₃)-CH₂)_{b}-(O-CH₂-CH₂)ₐ-OH,

   formule dans laquelle a va de 2 à 120, et b va de 1 à 100.
   Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC^{®} comme les SYNPERONIC PE/ L44^{®} et SYNPERONIC PE/F127^{®} par la société ICI.
b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :
   - les esters et éthers d'oses tels que les stéarate de sucrose, cocoate de sucrose, stéarate de sorbitan et leurs mélanges comme l'Arlatone 2121^{®} commercialisé par la société ICI ;
   - les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M^{®} par la société GOLDSCHMIDT, laurate de glycéryle tel que le produit vendu sous la dénomination IMWITOR 312^{®} par la société HULS, stéarate de polyglycéryl-2, tristéarate de sorbitan, ricinoléate de glycéryle ;
   - le mélange de cyclométhicone/diméthicone copolyol vendu sous la dénomination Q2-3225C^{®} par la société DOW CORNING.
- c) Les tensioactifs anioniques tels que :
   - les sels d'acides gras en C16-C30 notamment ceux dérivant des amines, comme le stéarate de triéthanolamine et/ou le stéarate d'amino-2-méthyl-2-propane di-ol-1,3 ;
   - les sels d'acides gras polyoxyéthylénés notamment ceux dérivant des amines ou les sels alcalins, et leurs mélanges ;
   - les esters phosphoriques et leurs sels tels que le "DEA oleth-10 phosphate" (Crodafos N 10N de la société CRODA) ou le phosphate de monocétyle monopotassique (Amphisol K de Givaudan) ;
   - les sulfosuccinates tels que le "Disodium PEG-5 citrate lauryl sulfosuccinate" et le "Disodium ricinoleamido MEA sulfosuccinate" ;
   - les alkyléthersulfates tels que le lauryl éther sulfate de sodium;
   - les iséthionates ;
   - les acylglutamates tels que le "Disodium hydrogenated tallow glutamate" (AMISOFT HS-21 R^{®} commercialisé par la société AJINOMOTO) et leurs mélanges.

Convient tout particulièrement à l'invention, le stéarate de triéthanolamine. Ce dernier est généralement obtenu par simple mélange de l'acide stéarique et de la triéthano lamine.

Chacune des compositions (i) ou (ii) conformes à l'invention peut également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100^{®} par la société PHOENIX CHEMICAL.

### Huiles

Chacune des compositions (i) et (ii) conforme à l'invention peut comprendre une ou plusieurs huiles ou solvant organique.

Par huile ou solvant organique, on entend un corps non aqueux liquide à température ambiante et pression atmosphérique. L'huile peut être volatile ou non volatile.

Par " huile ou solvant organique volatile", on entend au sens de l'invention tout milieu non aqueux susceptible de s'évaporer au contact des cils en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par "huile non volatile", on entend une huile restant sur les cils à température ambiante et pression atmosphérique au moins plusieurs heures et ayant notamment une pression de vapeur inférieure à 10⁻³ mm de Hg (0,13Pa).

L'huile peut être présente dans la composition dans une teneur allant de 1 à 30 %, de préférence 1 à 15 % en poids %, en poids par rapport au poids total de la composition. La composition (i) ou (ii) peut comprendre des huiles volatiles et/ou des huiles non volatiles, et leurs mélanges.

Les huiles (ou solvants organiques) volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars^{®}" ou de "Permetyls^{®}", les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination "Shell Solt^{®}" par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes (6.10⁻⁶ m²/s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants organiques volatils notamment fluorés tels que le nonafluorométhoxy butane ou le perfluorométhylcyclopentane.

Chacune des compositions (i) et (ii) conformes à l'invention peut également comprendre au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STÉARINERIES DUBOIS ou ceux vendus sous les dénominations de Miglyol 1810^{®}, 812^{®} et 818^{®} par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ; et leurs mélanges.

Les huiles de silicone non volatiles utilisables dans l'une ou l'autre des compositions (i) ou (ii) conformes à l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'une ou l'autre des compositions (i) ou (ii) conformes à l'invention sont notamment des huiles fluorosiliconées, des polyéthers fluorés, des silicones fluorées telles que décrit dans le document EP-A-847752.

La teneur en huile ou solvant organique non volatile dans l'une ou l'autre des compositions (i) ou (ii) conformes à l'invention peut varier de 0,01 à 30 % en poids, en particulier de 0,1 à 25 % en poids, et mieux de 0,1 à 20 % par rapport au poids total de la composition.

### Matière colorante

Chacune des compositions (i) ou (ii) conformes à l'invention peut également comprendre au moins une matière colorante comme les matières pulvérulentes, les colorants liposolubles, les colorants hydrosolubles.

Selon un mode de réalisation, les compositions (i) et (ii) présentent la même teinte.

Selon un autre mode de réalisation, les compositions (i) et (ii) présentent des couleurs différentes, de manière à renforcer l'effet maquillage obtenu.

On peut par exemple appliquer une composition de couleur claire sur l'ensemble de la frange de cils ou une portion interne de la frange de cils et une composition de couleur plus foncée sur la partie externe de la frange de cils.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de chaque composition (i) et (ii).

### Charges

Chacune des compositions (i) ou (ii) conformes à l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination Orgasol^{®} par la société ATOCHEM, de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel^{®} par la société NOBEL INDUSTRIE, les poudres acryliques telles que celles commercialisées sous la dénomination Polytrap^{®} par la société DOW CORNING, les particules de polyméthacrylate de méthyle et les microbilles de résine de silicone (Tospearls^{®} de TOSHIBA, par exemple), le carbonate de calcium précipité, le carbonate et l'hydro-carbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel^{®} 820 DU 40 et Expancel^{®}007WU par la Société AKZO NOBEL.

Les charges peuvent représenter de 0,1 à 25 %, en particulier de 1 à 20 % en poids par rapport au poids total de chaque composition.

Chacune des compositions (i) ou (ii) conformes à l'invention peut comprendre, en outre, tout additif usuellement utilisé en cosmétique tels que les antioxydants, les conservateurs, les fibres, les parfums, les neutralisants, les gélifiants, les épaississants, les vitamines, les agents de coalescence, les plastifiants, et leurs mélanges.

### Fibres

Chacune des compositions (i) ou (ii) conformes à l'invention peut en outre comprendre des fibres qui permettent une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, en particulier de 0,1 mm à 5 mm et plus particulièrement de 0,3 mm à 3,5 mm. Leur section peut être comprise dans un cercle de diamètre allant de 2 nm à 500 µm, en particulier allant de 100 nm à 100 µm et plus particulièrement de 1 µm à 50 µm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL^{®}, KERMEL TECH^{®} par la société RHODIA ou de poly-(p-phénylènetéréphtalamide) (ou d'aramide) notamment vendues sous la dénomination Kevlar ® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

### Actifs cosmétiques

Comme actifs cosmétiques pouvant être utilisés dans les compositions (i) ou (ii) conformes à l'invention, on peut citer notamment des antioxydants, les conservateurs, les parfums, les neutralisants, émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

Selon un mode particulier de réalisation de l'invention :
- la composition (i) comprend une phase aqueuse continue, au moins une cire dite collante, telle que décrite plus haut, possédant un collant supérieur ou égal à 0,7 N.s et une dureté inférieure ou égale à 3,5 MPa, au moins l'association d'un polymère hydrophile cationique et d'un polymère hydrophile anionique et au moins un terpolymère acrylique particulier tel que décrit plus haut, et
- la composition (ii) comprend une phase aqueuse continue, au moins une cire dite collante, telle que décrite plus haut, possédant un collant supérieur ou égal à 0,7 N.s et une dureté inférieure ou égale à 3,5 MPa, au moins l'association d'un polymère hydrophile cationique et d'un polymère hydrophile anionique et au moins un polymère filmogène en dispersion dans une phase aqueuse

Les deux compositions (i) et (ii) peuvent également différer par au moins une caractéristique optique visible à l'oeil nu, autre que la teinte procurée par la présence de matières colorantes. Il peut notamment s'agir de la brillance.

### KIT

Le kit selon la présente invention peut avantageusement comprendre un ou plusieurs moyens d'application des compositions cosmétiques (i) et (ii) conformes à l'invention.

La composition (ii) est appliquée à l'aide du moyen d'application décrit plus particulièrement ci-après.

La composition (i) peut être appliquée à l'aide de toute brosse ou tout peigne communément employé pour le maquillage des cils et/ou des sourcils.

En l'occurrence, il est particulièrement avantageux de procéder à l'application de la composition (i) avec une brosse de maquillage telle que décrite dans les brevets FR 2 701 198, FR 2 605 505, EP 792 603 et EP 663 161.

Le kit de maquillage selon l'invention peut, selon un mode de réalisation particulier, comprendre au moins deux conditionnements distincts, l'un comprenant la composition (i) définie plus haut et l'autre comprenant la composition (ii) également définie plus haut, l'une et l'autre pouvant indifféremment être une composition "topcoat" ou "basecoat" suivant l'ordre d'application comme cela a été décrit plus haut.

Le kit selon l'invention peut comporter un organe d'essorage. Cet organe d'essorage peut par exemple comporter un bloc d'un matériau alvéolaire tel qu'une mousse à cellules ouvertes ou fermées, avec ou sans flocage. En variante, l'organe d'essorage peut comporter un matériau non alvéolaire, éventuellement floqué, par exemple un élastomère ou une polyoléfine. Dans ce cas notamment, l'organe d'essorage peut par exemple comporter au moins une fente et/ou comporter une lèvre agencée pour essorer la tige.

Le maquillage des cils ou des sourcils étant effectué grâce à un geste multiple de l'utilisatrice, à savoir au moins en deux étapes, la première consistant en l'application de la composition "basecoat" et la deuxième consistant en l'application de la composition "topcoat" en tout ou partie sur ladite composition cosmétique, un kit de maquillage conditionné dans un seul et même conditionnement est particulièrement adapté. Cette alternative constitue un mode de réalisation préféré de l'invention.

Lorsque le kit est sous forme d'un seul et même conditionnement, il peut se présenter comme un récipient délimitant au moins un compartiment ou réservoir qui comprend la composition (i), ledit compartiment étant fermé par un élément de fermeture et au moins un compartiment ou réservoir qui comprend la composition (ii), étant également fermé par un élément de fermeture.

Toujours lorsque le kit est sous forme d'un seul et même conditionnement, celui-ci comprend de préférence au moins un moyen d'application ou applicateur pour la composition (i), notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient, comportant avantageusement deux compartiments ou réservoirs, peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient est de préférence équipé d'un essoreur disposé au voisinage d'au moins une ouverture du récipient. Un tel essoreur permet d'essuyer l'applicateur et éventuellement, la tige dont il peut être solidaire. Un tel essoreur est décrit par exemple dans le brevet FR 2 792 618.

Le contenu des brevets ou demandes de brevets cités précédemment, sont incorporés par référence dans la présente demande.

Selon un mode de réalisation particulièrement préféré, le kit de maquillage comprend deux réservoirs comprenant chacun l'une des compositions "basecoat" et "topcoat", l'un des réservoirs étant muni d'une brosse de maquillage, notamment de type brosse mascara telle que décrite ci-dessus et l'autre réservoir étant muni du moyen d'application de la composition (ii) décrit ci-après.

### MOYEN D'APPLICATION

Le moyen d'application compris dans le kit selon la présente invention comporte une pluralité d'éléments d'application disposés sur un support sous forme d'au moins une rangée dans la longueur est telle que les éléments d'application puissent au mieux contacter simultanément au plus un quart des cils de la frange.

Selon un mode de réalisation préféré, le moyen d'application compris dans le kit selon l'invention, comporte :
- une tige,
- un support relié à la tige et s'étendant selon un axe longitudinal, et
- une pluralité d'éléments d'application, au nombre de deux seulement le cas échéant, disposés sur le support et s'étendant sensiblement transversalement à l'axe longitudinal du support.

Ainsi, selon un mode de réalisation tout particulièrement préféré, le moyen d'application comporte :
- une pluralité d'éléments d'application (6 ; 60) disposés sur un support (5) sous forme d'au moins une rangée dont la longueur est telle que les éléments d'application (6 ; 60) puissent au mieux contacter simultanément au plus un quart des cils de la frange.

Les éléments d'application peuvent s'étendre sur une distance le long de l'axe longitudinal du support qui est telle que les éléments d'application puissent au mieux contacter simultanément au plus un quart, voire un cinquième ou un sixième des cils ou des sourcils de la frange, alors que l'axe longitudinal du support est orienté sensiblement tangentiellement à la frange.

Une portion de cils ou de sourcils maquillés peut par exemple correspondre à au plus un quart, voire un cinquième ou un sixième de la longueur de la frange.

Les éléments d'application peuvent comporter des dents et/ou des poils.

Cet applicateur permet de charger convenablement les cils tout en exerçant une action de peignage éventuelle.

Les éléments d'application s'étendent sur le support sur une distance suffisamment courte pour pouvoir maquiller une ou plusieurs mèches de cils, ce qui permet l'obtention de nouveaux effets de maquillage.

Cet applicateur permet de déposer de façon soutenue de la matière sur seulement au plus un tiers, voire un quart de la frange de cils à maquiller, de préférence la portion se trouvant à l'extérieur de la frange de cils.

Les éléments d'application peuvent par exemple s'étendre sur le support sur une distance comprise entre environ 0,1 et 1 cm, par exemple inférieure ou égale à 8 mm, voir 7 mm ou 6 mm, par exemple une distance allant de 2 mm à 5 mm environ, par exemple entre 2 mm et 3 mm.

L'applicateur compris dans le kit selon la présente invention peut comporter :
- une tige,
- un support relié à la tige et s'étendant selon un axe longitudinal,
- au moins une rangée d'éléments d'application, notamment constituée par des dents, disposées sur le support, les éléments d'application de la rangée s'étendant chacun sensiblement transversalement à l'axe longitudinal du support et présentant des extrémités libres dont la distance à l'axe longitudinal varie de manière non monotone lorsque l'on se déplace entre les éléments d'application extrêmes de la rangée.

La présence d'un maximum dans la rangée d'éléments d'application peut permettre une pénétration progressive des éléments d'application dans les cils et faciliter ainsi le maquillage d'une portion de frange de cils au sens de l'invention.

Le nombre d'éléments d'application de la rangée, notamment le nombre de dents, peut être compris par exemple entre 3 et 9, de préférence entre 3 et 8, notamment entre 3 et 7.

Selon un mode de réalisation, le support et les éléments d'application sont réalisés d'une seule pièce, notamment par moulage ou usinage. En variante, les éléments d'application peuvent être rapportés sur le support.

Le support et la tige peuvent être réalisés d'une seule pièce, notamment par moulage ou, en variante, le support peut être rapporté sur la tige. Dans ce cas, le support peut comporter un embout agencé pour s'engager dans un logement de la tige ou la tige peut comporter un embout agencé pour s'engager dans un logement du support.

La tige peut s'étendre selon un axe longitudinal distinct de l'axe longitudinal du support. Dans ce cas, l'axe longitudinal de la tige peut former avec l'axe longitudinal du support, notamment pour la portion de celui-ci qui porte les éléments d'application, un angle compris entre 5° et 45°, notamment compris entre 15° et 25°, par exemple égal à 20° environ. Cette configuration peut permettre une manipulation plus confortable de l'applicateur lors de l'application d'une composition de maquillage conforme à l'invention sur les cils. L'axe longitudinal du support peut être par exemple curviligne ou rectiligne.

La tige peut, en variante, s'étendre selon un axe longitudinal confondu avec l'axe longitudinal du support.

L'applicateur et/ou le support peuvent être réalisés au moins partiellement en un matériau souple ou rigide, notamment au moins partiellement en matière thermoplastique, élastomère ou thermoplastique élastomère, notamment par moulage. On ne sort pas du cadre de la présente invention si l'applicateur est réalisé au moins partiellement en bois ou en métal ou en d'autres matériaux encore, notamment par usinage.

Lorsque le support est réalisé dans un matériau élastiquement déformable, cela peut permettre de réaliser un essorage préférentiel d'un côté du support.

La tige et le support peuvent être ou non réalisés dans des matériaux différents.

Lorsque l'applicateur est observé de côté, une ligne reliant les extrémités libres des éléments d'application peut présenter une distance à l'axe longitudinal du support qui passe par au moins un extremum, notamment un maximum, ce qui peut rendre plus progressive la pénétration de l'applicateur dans les cils.

La ligne précitée peut notamment présenter au moins partiellement une forme pointue, notamment triangulaire, ou arrondie, notamment circulaire ou ogivale.

Quel que soit le profil de la ligne précitée, lorsqu'il y a un maximum et un minimum, l'angle formé entre d'une part la droite reliant les extrémités libres d'un élément d'application de plus grande longueur associé à ce maximum et d'un élément d'application de plus petite longueur associé à un minimum le plus proche de l'élément d'application de plus grande longueur, et d'autre part la normale à l'axe longitudinal du support, cette normale passant par l'extrémité libre de l'élément d'application de plus grande longueur, est par exemple compris entre 25° et 60° environ, notamment entre 25° et 50°, notamment entre 25° et 45°, par exemple entre 30° et 45°, par exemple égal à 43° environ.

La distance de la ligne précitée à l'axe longitudinal du support peut encore passer par deux extrema, voire plus.

Les éléments d'application peuvent être disposés selon une seule rangée ou selon au moins deux rangées. Dans ce dernier cas, les éléments d'application d'une première rangée et ceux d'une deuxième rangée peuvent s'étendre dans des directions différentes ou, en variante, dans des directions parallèles. Les deux rangées peuvent par exemple être disposées sensiblement à l'opposé l'une de l'autre relativement au support.

La hauteur des éléments d'application peut par exemple être comprise entre 0,5 et 10 mm environ, notamment entre 1 et 3 mm environ.

Lorsque les éléments d'application sont disposés selon au moins une rangée, cette dernière peut comporter une succession d'éléments d'application s'étendant alternativement, au moins partiellement, de part et d'autre d'une surface géométrique de séparation.

Les éléments d'application peuvent présenter des bases alignées ou non.

Lorsque les éléments d'application sont disposés selon au moins une rangée, la rangée peut s'étendre selon un axe non rectiligne. Les éléments d'application peuvent également être réalisés alternativement de part et d'autre d'une âme médiane.

Toujours lorsque les éléments d'application sont disposés selon au moins une rangée, cette rangée peut comporter une succession d'éléments d'application situés alternativement au moins partiellement de part et d'autre d'une surface géométrique de séparation, deux éléments d'application consécutifs ayant des parties adjacentes jointives ou se chevauchant lorsque l'applicateur est observé de côté et formant entre eux une échancrure permettant d'y recevoir un cil.

L'applicateur peut comporter un peigne ou une brosse.

Lorsque l'applicateur comporte une brosse, le support peut comporter deux fils torsadés de petit diamètre, notamment inférieur à 0,7 mm, par exemple compris entre 0,2 et 0,6 mm, mieux entre 0,35 et 0,5 mm.

Toujours dans le cas où l'applicateur comporte une brosse, les éléments d'application peuvent être des poils de petit diamètre, par exemple inférieur ou égal à 8/100 mm, mieux 6/100 mm.

Lorsque la brosse comporte une âme torsadée, le nombre de spires de la brosse peut être compris entre 3 et 6, par exemple. Dans le cas d'une brosse à âme torsadée, les poils s'étendent à partir de l'âme selon deux nappes hélicoïdales. Le nombre de spires de la brosse correspond à la somme des révolutions autour de l'âme des deux nappes. Une partie de l'âme torsadée peut être dépourvue de poils, notamment à proximité de la jonction entre le support et la tige.

La section transversale de la surface enveloppe de la brosse peut être circulaire, carrée, ou autre.

La brosse peut présenter une surface enveloppe biconique. La surface enveloppe peut encore être conique ou tronconique avec la base du cône située du côté de l'extrémité distale ou proximale de la brosse.

L'applicateur compris dans le kit selon l'invention, peut prendre la forme d'une brosse pour l'application d'un produit sur les cils, comportant une âme torsadée et des poils s'étendant à partir de l'âme, le nombre de spires étant inférieur ou égal à 6.

L'applicateur compris dans le kit selon l'invention, peut encore prendre la forme d'une brosse pour l'application d'un produit sur les cils, comportant une âme et des poils s'étendant à partir de l'âme, la longueur de la portion de la brosse portant les poils étant inférieure ou égale à 8 mm, mieux à 7 mm voire à 6 mm.

L'applicateur compris dans le kit selon l'invention, peut encore prendre la forme d'une composition conforme à l'invention, comportant une âme et des poils s'étendant à partir de l'âme, contenus dans une surface enveloppe de forme sensiblement tronconique, conique ou biconique, dans laquelle le plus petit angle formé entre un plan contenant la plus grande section transversale de la brosse et une tangente à la surface enveloppe de la brosse à l'intersection avec ce plan est compris entre 20° et 60°, notamment compris entre 25° et 50°, par exemple entre 30° et 35°.

L'applicateur compris dans le kit selon l'invention, peut encore prendre la forme d'une composition conforme à l'invention sur les cils, comportant une âme et des poils s'étendant à partir de l'âme, dans laquelle la surface enveloppe de la brosse présente une forme sensiblement biconique, d'angle au sommet à la jonction des troncs de cônes inférieur ou égal à 120°, voire inférieur ou égal à 90°.

L'applicateur compris dans le kit selon l'invention, peut encore prendre la forme d'un peigne pour l'application d'un produit sur les cils, comportant un support et des dents s'étendant transversalement à l'axe longitudinal du support, la distance entre les dents extrêmes le long de l'axe longitudinal étant inférieure ou égale à 8 mm, mieux 7 mm, voire 6 mm.

L'applicateur compris dans le kit selon l'invention, peut encore prendre la forme d'un peigne pour l'application d'un produit sur les cils, comportant un support et des dents s'étendant transversalement à l'axe longitudinal du support, comportant une dent de plus grande longueur ayant une première longueur et une dent de plus petite longueur ayant une deuxième longueur, peigne dans lequel le rapport de la première longueur à la deuxième longueur est supérieur ou égal à 1,3.

L'applicateur compris dans le kit selon l'invention, peut encore prendre la forme d'un peigne pour l'application d'un produit sur les cils, comportant un support et des dents s'étendant transversalement à l'axe longitudinal du support, dans lequel l'angle formé entre une droite joignant l'extrémité libre d'une dent de plus petite longueur et l'extrémité libre d'une dent de plus grande longueur et la perpendiculaire à l'axe longitudinal du support passant par l'extrémité libre de la dent de plus grande longueur est compris entre 20° et 60°.

Lorsque le kit comporte un organe d'essorage, le support de l'applicateur est profilé de façon à faciliter le passage dans l'organe d'essorage et peut comporter par exemple à cet effet à l'avant un nez arrondi et à l'arrière un bossage.

La tige de l'applicateur peut être reliée à une capsule de fermeture du récipient pouvant également avoir un rôle d'organe de préhension et l'applicateur peut être logé à l'intérieur du récipient lorsqu'il est en position fermée.

L'invention pourra être mieux comprise à la lecture de la description détaillée qui va suivre, d'exemples de mise en oeuvre non limitatifs du moyen d'application compris dans le kit et de kits en tant que tels, et à l'examen du dessin annexé, sur lequel :
- la figure 1 est une vue schématique en élévation avec coupe partielle d'une partie du kit, lorsque le kit se présente sous la forme de deux conditionnements distincts, à savoir du conditionnement comprenant le moyen d'application conforme à l'invention, ainsi que l'une des deux compositions conformes à l'invention,
- la figure 2 illustre l'utilisation d'un applicateur conforme à l'invention,
- la figure 3 est une vue de côté, schématique et partielle, de l'applicateur de la figure 1,
- la figure 4 est une vue en perspective, schématique et partielle, de l'applicateur de la figure 1,
- la figure 5 est une vue analogue à la figure 3, d'une variante d'applicateur,
- la figure 6 est une vue analogue à la figure 4, représentant l'applicateur de la figure 5,
- les figures 7 à 9 représentent en vue de côté, schématique et partielle, d'autres variantes de l'applicateur de la figure 1,
- les figures 10 et 11 représentent de manière schématique, avec coupe partielle, des exemples de fixation du support sur la tige de l'applicateur,
- la figure 12 représente en vue de côté, schématique et partielle, une variante de réalisation de l'applicateur,
- les figures 13 à 17 représentent en vue de côté, de manière schématique et partielle, différents profils d'applicateurs conformes à l'invention,
- la figure 18 représente en vue de côté, schématique et partielle, une variante d'applicateur comportant deux rangées d'éléments d'application,
- les figures 19 à 22 représentent en coupe axiale, schématique, différents applicateurs comportant une ou plusieurs rangées d'éléments d'application,
- les figures 23 et 24 représentent en vue de côté différentes configurations d'éléments d'application,
- la figure 25 représente une variante de la partie du kit de la figure 1 comportant un autre d'organe d'essorage,
- les figures 26 et 27 représentent en coupe axiale, schématique et partielle, d'autres exemples encore d'organes d'essorage pouvant être utilisés dans un kit selon l'invention,
- la figure 28 est une vue analogue à la figure 1 d'une variante comportant une brosse,
- les figures 29 à 35 sont des vues schématiques et partielles de variantes de brosses,
- la figure 36 est une coupe transversale schématique de la brosse de la figure 28,
- la figure 37 est une vue en perspective, partielle, d'un exemple de réalisation dans lequel une composition conforme à l'invention est déposée sur l'élément d'application à partir d'un tube flexible,
- les figures 38 à 42 sont des coupes axiales, schématiques, de kits selon l'invention sous forme d'un seul et même conditionnement, et
- la figure 43 illustre le type de maquillage que l'on peut obtenir selon le procédé de maquillage de l'invention.

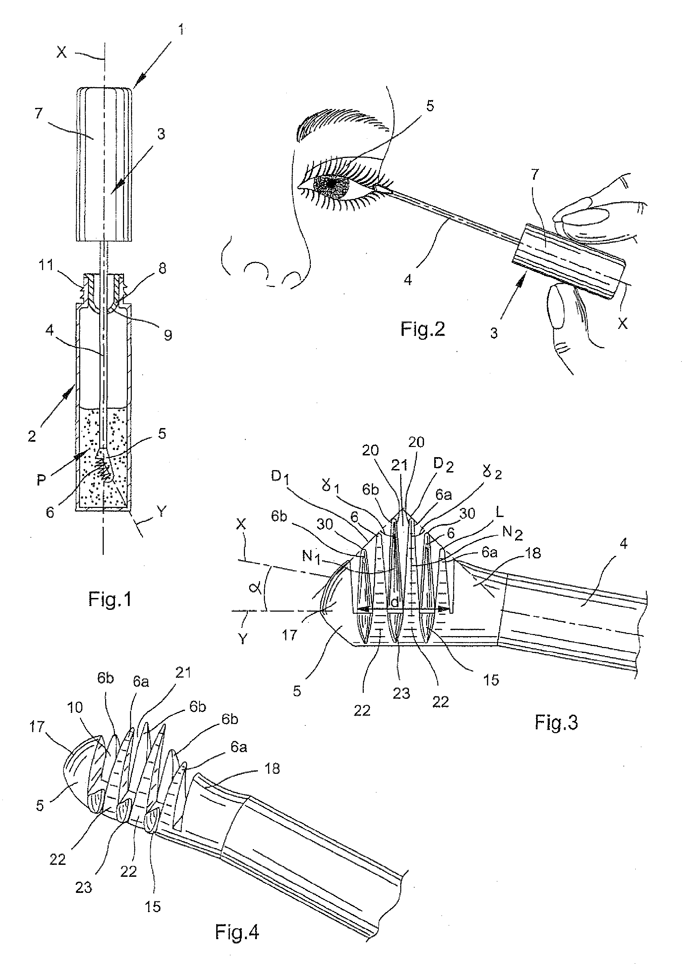

Le dispositif 1, représenté sur la figure 1, qui correspond à une partie du kit lorsque ce dernier est conditionné dans deux conditionnements distincts comporte un récipient 2 contenant une réserve d'un produit cosmétique P, en l'occurrence l'une des compositions (i) ou (ii), et un applicateur 3 comportant une tige 4 d'axe longitudinal X. Seul le dispositif comprenant le moyen d'application conforme à l'invention et l'une des compositions conforme à l'invention, est ici représenté

La tige 4 est munie à une extrémité d'un support 5 comportant une pluralité d'éléments d'application 6 et la tige 4 se raccorde à l'extrémité opposée à une capsule de fermeture 7 du récipient 2 qui constitue également un élément de préhension.

La capsule de fermeture 7 comporte un filetage intérieur non apparent, destiné à se visser sur le col 11, fileté extérieurement, du récipient 2.

Le récipient 2 comporte, dans l'exemple décrit, un organe d'essorage 8 engagé dans le col 11 et comportant une lèvre d'essorage 9 agencée pour essorer la tige 4 et le support 5 muni des éléments d'application 6.

L'organe d'essorage 8 peut, le cas échéant, être agencé de telle sorte qu'il puisse se déformer au passage des éléments d'application 6.

L'organe d'essorage 8 peut être réalisé par moulage par injection, par exemple dans une polyoléfine.

Le récipient 2 est fermé de façon étanche en l'absence d'utilisation par vissage de la capsule de fermeture 7 sur le col 11.

Dans l'exemple représenté, l'applicateur 3 comporte un peigne, les éléments d'application 6 étant constitués par des dents. Le support 5 s'étend selon un axe longitudinal Y formant avec l'axe longitudinal X de la tige 4 un angle α non nul, tel qu'illustré sur la figure 3 notamment. Cet angle α peut être compris entre 5° et 45° par exemple.

Les éléments d'application 6, au nombre de six par exemple, sont disposés dans l'exemple considéré selon une rangée 10.

Les éléments d'application 6 peuvent se situer sur le support 5 selon diverses configurations.

Dans l'exemple illustré, la rangée 10 comporte une première série de dents 6a et une deuxième série de dents 6b, respectivement situées de part et d'autre d'une surface géométrique de séparation S, qui est ici un plan médian de symétrie du peigne, parallèle au plan de la figure 3, et peut constituer également un plan de joint pour le moulage du peigne.

Dans l'exemple illustré, les dents 6a et 6b sont réalisées alternativement de part et d'autre d'une âme commune 15 du support 5, qui sert en quelque sorte de squelette sur lequel les dents se raccordent en partie inférieure.

Le support 5 est pourvu à l'avant d'un nez arrondi 17, destiné à faciliter sa rentrée dans le récipient 2.

Le support 5 comporte, à l'arrière, un bossage 18 destiné à faciliter la traversée de l'organe d'essorage 8 lors de son extraction du récipient 2.

Les éléments d'application 6 comportent chacun une partie supérieure 20 se terminant par une extrémité libre 30 et une partie inférieure 22 reliée à l'âme 15 du support 5.

Dans l'exemple considéré, les éléments d'application 6 s'étendent sensiblement perpendiculairement à l'axe Y du support 5.

Les extrémités libres 30 des éléments d'application se situent à une distance à l'axe longitudinal Y variant de manière non monotone lorsque l'on se déplace entre les éléments d'application extrêmes de la rangée 10, soit de gauche à droite sur la figure 3.

Par "variant de manière non monotone" au sens de la présente invention, il faut comprendre que la ligne reliant les extrémités libres des éléments d'application présente une distance à l'axe longitudinal du support qui passe par au moins un extremum, par exemple un maximum.

Dans l'exemple illustré, la ligne *L* reliant les extrémités libres 30 présente, lorsque l'applicateur est observé de côté, dans une direction perpendiculaire à l'axe longitudinal de la tige 4 et à la surface géométrique de séparation précitée, comme illustré sur la figure 3, une forme sensiblement triangulaire, avec deux segments rectilignes formant entre eux un angle sensiblement égal à 90° sur le dessin.

Dans l'exemple considéré, la succession d'éléments d'application 6 comporte deux plus grandes dents 6a et 6b de même longueur.

La droite D₁ passant par l'extrémité libre de l'élément de plus petite longueur en partant de l'extrémité distale de l'applicateur et par l'extrémité libre de l'élément de plus grande longueur le plus proche de cet élément de plus petite longueur est confondue dans l'exemple considéré avec le premier segment rectiligne de la ligne L.

L'angle γ₁ formé entre la droite D₁ et la perpendiculaire N₁ à l'axe Y passant par l'extrémité libre de l'élément d'application de plus grande longueur précité est compris par exemple entre 25° et 60°, étant par exemple d'environ 43°.

La droite D₂, passant par le plus petit élément d'application à partir de l'extrémité proximale du support 5 et le plus grand élément d'application le plus proche de cet élément est confondue avec le deuxième segment rectiligne de la ligne L.

L'angle γ₂ formé entre la droite D₂ et la perpendiculaire N₂ à l'axe Y passant par l'extrémité libre de ce plus grand élément d'application peut être compris dans les mêmes gammes de valeur que l'angle γ₁, étant par exemple sensiblement égal à γ₁. L'angle au sommet entre les segments de la ligne L est par exemple inférieur ou égal à 120°, voire inférieur ou égal à 90°.

Les éléments d'application 6 s'étendent sur le support 5 sur une distance d relativement faible, notamment une distance d comprise entre 0,1 et 1 cm environ. Ainsi, comme illustré sur la figure 2, on peut, à l'aide de l'applicateur 3, maquiller seulement une portion de la frange de cils à la fois sans atteindre la totalité des cils de la paupière. Par exemple, les éléments d'application peuvent contacter simultanément, au maximum, moins d'un quart des cils de la paupière supérieure, comme illustré.

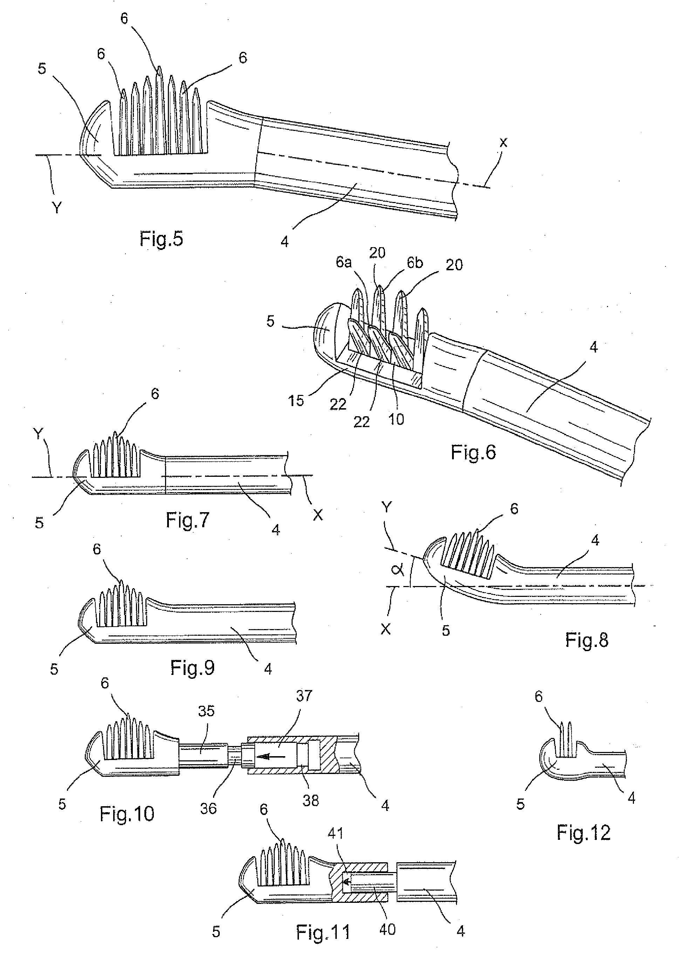

Le support 5 et les éléments d'application 6 sont réalisés d'une seule pièce dans l'exemple illustré, par moulage de matière plastique. Le support 5 est rapporté sur la tige 4 d'une manière qui sera détaillée plus loin, au regard des figures 10 et 11.

Dans l'exemple décrit, les parties supérieures 20 de deux éléments d'application 6 consécutifs ménagent entre eux une échancrure 21 s'étendant sensiblement jusqu'à l'âme 15, lorsque le peigne est observé de côté, comme sur la figure 3. Les échancrures 21 permettent de saisir plus facilement les cils, de manière par exemple à les séparer, les peigner ou lisser du produit déposé à leur surface.

On comprend qu'en jouant sur la longueur des éléments d'application et sur l'intervalle entre eux, on peut agir aisément sur la quantité de produit dont se charge l'applicateur.

L'écart entre les sommets de deux éléments d'application successifs 6a ou 6b situés d'un même côté de la surface géométrique de séparation est dans l'exemple considéré nettement supérieur à la largeur d'un élément d'application, mesuré à mi-hauteur environ de l'applicateur 3, parallèlement à l'axe Y du support 5.

Chaque élément d'application 6a s'étend sensiblement à mi-distance, lorsque l'applicateur 3 est observé de côté, de deux éléments d'application 6b adjacents et inversement.

Dans l'exemple considéré, les parties supérieures 20 des éléments d'application 6 pointent sensiblement toutes dans la même direction.

Les parties inférieures 22 de deux éléments d'application successifs situés d'un même côté de l'âme 15 forment entre elles et avec l'âme 15 une cavité 23 pouvant constituer une réserve de produit.

L'écartement entre les éléments d'application 6a ou 6b peut être relativement grand au profit de la quantité de produit dont se charge l'applicateur sans pour autant que l'applicateur perde toute aptitude à agripper les cils, grâce au décalage des éléments d'application et au fait que les échancrures 21 formées par les parties supérieures 20 des éléments d'application restent suffisamment étroites.

Les éléments d'application 6 peuvent présenter de nombreuses configurations sans que l'on sorte du cadre de la présente invention, notamment des parties supérieures orientées différemment.

On ne sort pas du cadre de la présente invention lorsque les éléments d'application 6 sont agencés de manière différente sur le support 5.

On a représenté sur les figures 5 et 6 un autre exemple d'applicateur comportant une pluralité de dents 6, par exemple au nombre de sept, s'étendant selon une rangée 10. Les dents 6, ainsi qu'on peut le voir sur la figure 6, ont des bases alignées 22 tandis que les parties supérieures 20 de deux dents 6a et 6b consécutives divergent.

La figure 7 illustre la possibilité de réaliser la tige 4 avec un axe longitudinal X qui est confondu avec celui Y du support 5.

Sur la figure 8, le support 5 est représenté avec un axe longitudinal Y formant un angle α non nul avec l'axe longitudinal X de la tige 4, le sens de l'inclinaison du support sur la figure 8 étant opposé à celui du support des figures 1 à 4.

Le support 5 peut être réalisé d'une seule pièce avec la tige 4, par exemple par moulage de matière plastique ainsi qu'illustré sur la figure 9. Mais le support 5 peut encore être rapporté sur la tige 4.

Dans l'exemple représenté à la figure 10, le support 5 comporte un embout 35 sensiblement cylindrique comportant une gorge annulaire 36. La tige 4 comporte un logement 37 correspondant, muni d'un bourrelet annulaire 38 agencé pour s'encliqueter dans la gorge 36.

L'embout 35 pourrait encore être configuré pour être emmanché à force dans la tige 4.

Dans l'exemple de la figure 11, la tige 4 comporte un embout sensiblement cylindrique 40, agencé pour venir se loger dans un logement correspondant 41 du support 5.

L'applicateur 3 peut comporter un nombre de dents inférieur à six ou sept, et notamment comporter seulement deux dents 6 ainsi qu'illustré sur la figure 12. Les dents peuvent présenter ou non une même largeur.

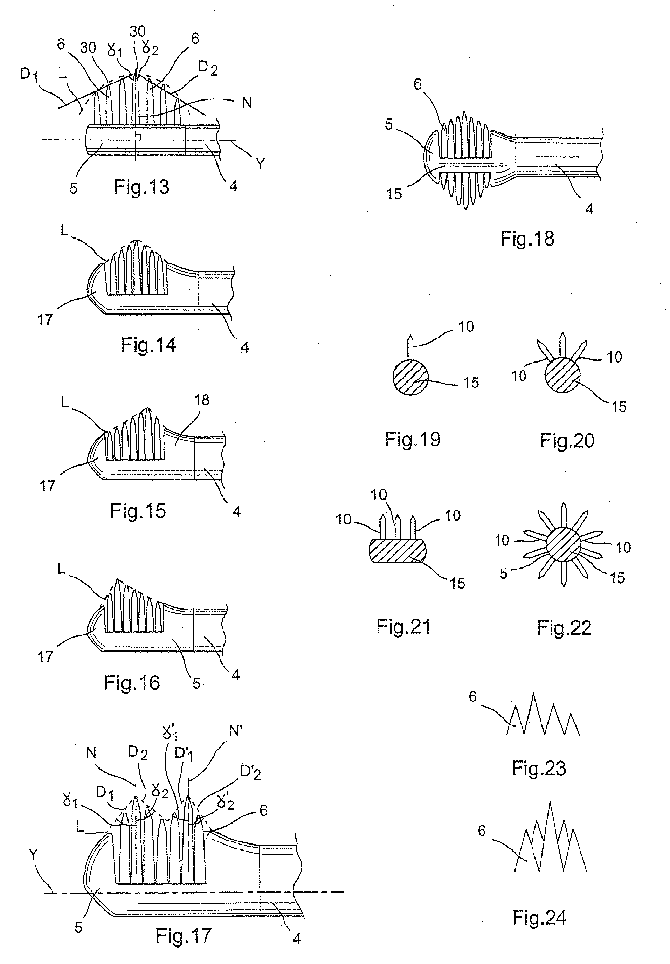

La ligne *L* reliant les extrémités libres 30 des éléments d'application 6 peut présenter diverses formes autres que celle des figures 1 à 11, comme illustré sur les figures 13 à 17.

En particulier, la ligne *L* peut présenter une forme arrondie, notamment sensiblement circulaire comme illustré sur la figure 13, ou ogivale comme illustré sur la figure 14. L'extremum peut être situé sensiblement au milieu du support 5 comme illustré précédemment, être plus proche de la tige 4 ainsi qu'illustré sur la figure 15 ou plus proche de l'extrémité distale de l'applicateur ainsi qu'illustré sur la figure 16. La distance de la ligne *L* à l'axe longitudinal Y du support 5 peut passer par deux extrema ainsi qu'illustré sur la figure 17.

On peut constater sur la figure 13 que le support 5 peut être dépourvu du nez arrondi 17 et/ou du bossage 18.

Sur cette figure, on a également représenté les droites D₁ et D₂ et la normale N à l'axe longitudinal Y du support 5 qui passe par l'extrémité libre de l'élément d'application de plus grande longueur, qui est unique dans l'exemple considéré.

Les angles γ₁ formés entre la droite D₁ et cette normale N et γ₂ formés entre la droite D₂ et cette normale N peuvent être égaux ou différents, étant par exemple compris dans les gammes de valeurs données précédemment.

Dans l'exemple de la figure 17, on peut définir des angles γ₁ et γ₂ relativement à la normale N passant par l'extrémité libre de l'élément d'application associé au premier extremum relatif et γ'1 et γ'2 relativement à la normale N' passant par l'extrémité libre de l'élément d'application correspondant au deuxième extremum relatif, ces angles étant là encore compris par exemple dans les gammes de valeurs données précédemment.

La figure 18 illustre la possibilité de réaliser sur le support 5 deux rangées d'éléments d'application, par exemple deux rangées situées à l'opposé l'une de l'autre relativement à l'âme 15 du support 5. Les éléments d'application peuvent s'étendre différemment d'une rangée à l'autre et notamment, ainsi qu'illustré sur la figure 18, présenter des profils différents lorsque l'applicateur 3 est observé de côté.

L'âme 15 peut présenter en section transversale une forme circulaire comme illustré sur les figures 19, 20 et 22 ou autre, par exemple aplatie, notamment sensiblement rectangulaire, comme illustré sur la figure 21.

Le support 5 peut comporter une rangée de dents alignées, comme illustré sur la figure 19, ou plusieurs rangées de dents s'étendant dans des directions différentes, par exemple divergentes comme illustré sur la figure 20 ou dans des directions parallèles comme illustré sur la figure 21.

On a représenté à titre illustratif sur la figure 22 un applicateur comportant une pluralité de rangées 10, disposées sur tout le pourtour de l'âme 15 du support 5.

Les éléments d'application 6 peuvent présenter, lorsque l'applicateur est observé de côté, sur au moins une partie de leur longueur, un profil de largeur sensiblement constante, comme illustré sur les figures 1 à 22. Les éléments d'application 6 peuvent encore présenter un profil différent, notamment sensiblement triangulaire, ainsi qu'illustré sur la figure 23, ou autre encore.

Les éléments d'application peuvent être disposés en quinconce, comme illustré sur la figure 24.

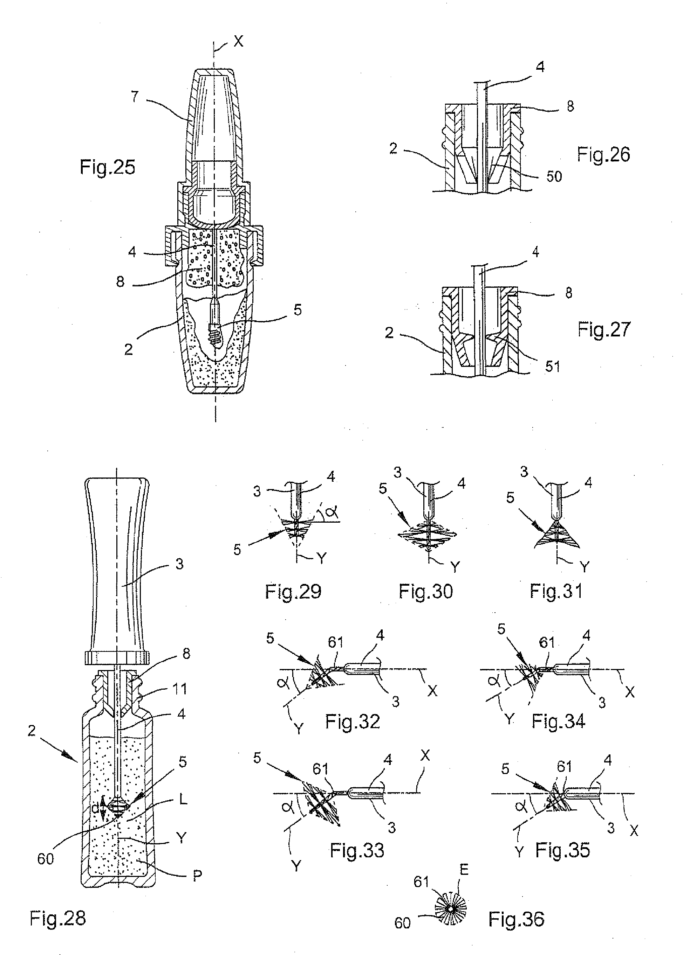

L'organe d'essorage 8 peut être différent de celui illustré à la figure 1 sans que l'on sorte du cadre de la présente invention. On a représenté, à titre d'exemple, à la figure 25 un dispositif dont l'organe d'essorage 8 est constitué par un matériau alvéolaire, notamment une mousse à cellules ouvertes ou fermées.

L'organe d'essorage 8 peut être réalisé, en variante, en élastomère et comporter au moins une fente 50 ainsi qu'illustré sur la figure 26. L'organe d'essorage 8 peut comporter une lèvre annulaire 51 ainsi qu'illustré sur la figure 27, ailleurs qu'à son extrémité inférieure.

Les éléments d'application peuvent être autres que des dents et comporter par exemple des poils, rapportés sur le support, comme illustré sur la figure 28. L'applicateur 3 comporte alors, par exemple, une brosse dont les poils 60 s'étendent sensiblement transversalement à l'axe longitudinal Y du support, qui peut être confondu avec l'axe longitudinal X de la tige 4 comme illustré.

Dans l'exemple considéré, les poils s'étendent sur une distance d le long de l'axe longitudinal Y du support qui est telle que les poils ne puissent pas contacter simultanément plus d'un quart des cils ou des sourcils de la frange de cils ou de sourcils que l'on souhaite maquiller. La distance d peut par exemple être comprise entre 2 et 3 mm environ, et le nombre de spires peut être compris entre 3 et 6, par exemple.

Lorsque l'applicateur 3 est observé de côté, comme illustré sur la figure 28, la ligne *L* reliant les extrémités libres des poils peut présenter une distance à l'axe Y qui passe par au moins un extremum.

Lorsque l'applicateur 3 comporte une brosse, les poils 60 utilisés sont de préférence relativement fins, par exemple de 6/100 mm environ de diamètre.

Les poils 60 peuvent présenter une section transversale pleine, sensiblement circulaire.

L'âme 61 peut être formée par un fil métallique plié en U et dont les branches sont torsadées sur elles-mêmes de manière à serrer entre elles les poils.

Le fil métallique utilisé est de préférence relativement fin, par exemple de diamètre inférieur à 0,7 mm, notamment compris entre 0,35 et 0,5 mm.

Les figures 29 à 35 représentent des variantes d'applicateurs.

Sur la figure 29, l'applicateur 3 comporte une brosse présentant une surface enveloppe sensiblement conique ou tronconique, les poils les plus longs se trouvant du côté de la tige 4.

L'angle δ formé entre la perpendiculaire à l'axe longitudinal Y de la brosse passant par la section transversale de plus grande dimension et la tangente à la surface enveloppe est par exemple compris entre 20° et 60°.

L'applicateur 3 de la figure 30 comporte une brosse de surface enveloppe sensiblement biconique, tandis que celui de la figure 31 comporte une brosse de surface enveloppe sensiblement conique ou tronconique, les poils les plus courts se trouvant du côté de la tige 4.

L'axe longitudinal Y de la brosse peut être aligné ou non avec celui X de la tige.

L'axe Y peut former un angle α avec l'axe X de la tige, comme illustré sur les figures 32 à 35. L'angle α peut par exemple être compris entre 5° et 45° environ, par exemple être égal à 20° environ.

La surface enveloppe E de la brosse peut être de section transversale circulaire comme illustré à la figure 36 ou autre, notamment polygonale, oblongue ou avec des crans ou créneaux.

Lorsque l'âme torsadée est coudée, le coude peut être situé plus ou moins près de la tige 4, comme illustré sur les figures 32 à 35.

Dans des variantes non illustrées, l'âme est courbe.

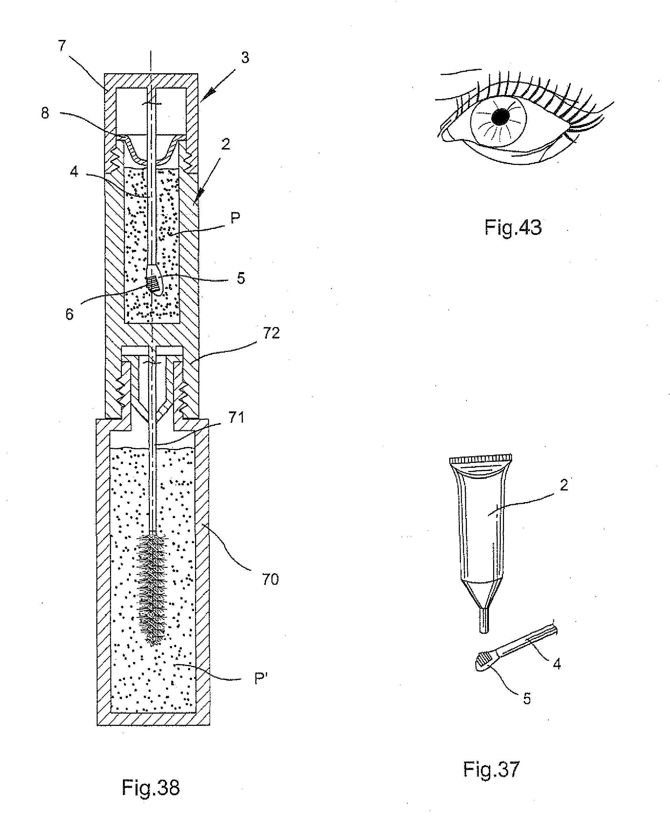

On a représenté sur la figure 37 une autre variante de mise en oeuvre de l'invention.

La figure 37 représente une partie du kit selon l'invention dans laquelle l'une des compositions conformes à l'invention est contenue dans un récipient 2 en forme de tube souple.

D'autres modes de distribution peuvent être utilisés pour déposer ladite composition sur l'applicateur 3, par exemple un distributeur à pompe, sans que l'on ne sorte du cadre de la présente invention.

La figure 38 représente un kit conformément à l'invention, sous forme d'un seul et même conditionnement, comportant un récipient 2 contenant une composition conforme à l'invention et l'applicateur 3, ainsi qu'un deuxième récipient 70 contenant la deuxième composition conforme à l'invention et un deuxième applicateur 71 qui peut être différent de l'applicateur 3. Par exemple, l'applicateur 71 peut comporter une brosse à mascara conventionnelle.

Dans l'exemple illustré à la figure 38, le récipient 2 comporte une jupe inférieure 72 munie d'un filetage et constituant une capsule de fermeture pour le récipient 70.

L'applicateur 71 peut permettre par exemple d'appliquer sur les cils la première composition, puis l'utilisateur peut utiliser l'applicateur 3 pour maquiller seulement une portion de la frange de cils, avantageusement au plus un tiers de ladite frange, et de façon encore plus préférée, la portion extérieure de la frange de cils, sur au plus un tiers de la frange.

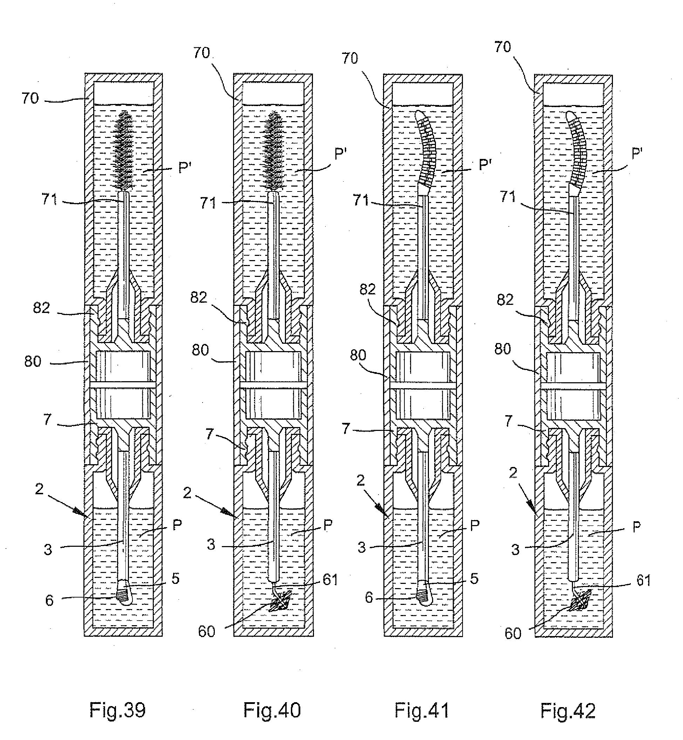

D'autres exemples de dispositifs comportant deux récipients et deux applicateurs ont été représentés sur les figures 39 à 42.

Les applicateurs sont dans ces exemples orientés dans des directions opposées de telle sorte que chaque récipient peut servir d'organe de préhension à l'applicateur associé à l'autre récipient.

Sur la figure 39, le dispositif comporte une brosse à mascara et un applicateur 3 conforme à l'invention comportant un peigne, tandis que l'applicateur 3 du dispositif de la figure 40 comporte une brosse. La brosse à mascara peut être choisie par exemple parmi celles décrites dans US 5 937 870 ou FR 2 605 505 par exemple, dont le contenu est incorporé à la présente demande par référence.

Le dispositif de la figure 41 comporte un peigne et un applicateur 3 conforme à l'invention, constitué par un peigne également. L'applicateur de la figure 42 comporte un peigne et un applicateur conforme à l'invention, constitué par une brosse. Le peigne des applicateurs 71 des figures 41 et 42 est par exemple choisi parmi ceux décrits dans US 6 581 610, WO 01/05271 ou US 6 539 950, dont le contenu est incorporé à la présente demande par référence.

Les dispositifs des figures 39 à 42 comportent un manchon 80 tubulaire reliant entre elles les capsules de fermeture 82 et 7 respectivement associées aux récipients 70 et 2. Les capsules de fermeture 82 et 7 peuvent être par exemple retenues par friction, collage ou encliquetage à l'intérieur du manchon 80.

La figure 43 représente un oeil maquillé selon le procédé de l'invention, à l'aide d'un kit selon l'invention. On constate que la portion extérieure de la frange de cil comporte davantage de matières déposées. On ne déplore pas la formation d'amas et un effet de maquillage particulier est obtenu.

Bien entendu, l'invention n'est pas limitée aux exemples de réalisation qui viennent d'être décrits. On peut notamment combiner entre elles les caractéristiques des différents modes de réalisation.

Dans des exemples de mise en oeuvre de l'invention, les éléments d'application peuvent s'étendre transversalement à la tige ou à l'organe d'essorage.

### PROCEDE

L'invention a pour objet un procédé de maquillage et/ou de soin non thérapeutique des cils, caractérisé en ce qu'il comprend :
- au moins une étape d'application sur les cils, d'au moins une couche d'une composition (i) présentant une teneur en cire(s) et en polymère(s) hydrophile(s) inférieure ou égale à 26 % en poids par rapport au poids total de la composition, et
- au moins une étape d'application d'au moins une couche d'une composition (ii), présentant une teneur totale en cire(s) et en polymère(s) filmogène(s) hydrophile(s) supérieure à 26 % en poids par rapport au poids total de la composition,
   au moins l'une desdites compositions (i) et (ii) comprenant une phase aqueuse continue, l'étape d'application de la composition (ii) étant effectuée à l'aide du moyen d'application décrit plus haut.

Selon un mode de réalisation préféré, la composition (i) est appliquée préalablement à la composition (ii). Avantageusement, la composition (ii) est de plus appliquée sur seulement une portion de la frange de cils. De cette façon, l'obtention du contraste en terme de différentiel de charge est optimale.

Ainsi, le procédé de maquillage peut comprendre les étapes consistant à :
- appliquer sur sensiblement l'ensemble de la frange de cils la première composition (i) conforme à l'invention, de manière à former un premier dépôt, et
- sur le premier dépôt de la portion formée par au plus le tiers extérieur de la frange de cils, former un second dépôt au moyen d'une seconde composition (ii) conforme à l'invention.

On peut par exemple former le second dépôt avant le séchage complet de la première composition.

La seconde portion peut s'étendre sur au moins 1/6^{ème} de la longueur totale de la frange de cils.

La frange maquillée peut être celle des cils de la paupière supérieure.

Selon un autre mode de réalisation particulier, la composition (i) est appliquée sur l'ensemble de la frange des cils et la composition (ii) est appliquée seulement sur au plus un tiers de la frange de cils, de préférence sur au plus le tiers extérieur de la frange de cils, voire sur au plus un quart de ladite frange. La portion de frange de cils sur laquelle la composition (i) peut être déposée peut représenter de 1/8 à 1/3, de préférence de 1/4 à 1/3 de la longueur totale de la frange de cils.

La présente invention est illustrée par les exemples qui suivent. Sauf indication contraire, les quantités indiquées sont exprimées en pourcentage massique par rapport au poids total de la composition.

Les polymères hydrophiles sont indiqués comme tels dans les exemples qui suivent.

### Exemples 1 à 4: compositions (i)

| | **Exemple 1** | **Exemple 2** | **Exemple 3** | **Exemple 4** |
|---|---|---|---|---|
| - Cire de carnauba | 7,3 | 2,9 | 5,35 | 3,5 |
| - Cire d'abeille | - | 3,7 | - | 7,4 |
| - Cire de candelilla | 2,5 | - | - | - |
| - Cire de paraffine | - | 11,8 | - | - |
| - Cire de son de riz | 7,45 | - | - | - |
| - Cire microcristalline | - | - | 5 | - |
| - Cire de polytetra fluoroethylène | - | 2 | - | - |
| - Cire obtenue par hydrogénation d'huile d'olive estérifiée avec l'alcool stéarylique (PHYTOWAX Olive 18 L 57 SOPHIM) | 6,3 | - | 6,45 | - |
| - (Hydroxystéaryloxy)stéarate d'alkyle en C18-C38 (Kester K82 P de Koster Keunen) | - | 0,2 | - | - |
| - Hydroxyéthyl cellulose quaternisée par chlorure de 2,3 époxypropyl triméthyl ammonium POLYMERE HYDROPHILE | - | 0,08 | - | - |
| - Hydroxyethylcellulose POLYMERE HYDROPHILE | 0,22 | 0,93 | 0,2 | - |
| - Polyméthacrylate de sodium dans l'eau à 25 % en MA* (Darvan 7 de Vanderbilt) POLYMERE HYDROPHILE | - | 0,9 | - | - |
| - Gomme arabique POLYMERE HYDROPHILE | 1,52 | 3,46 | - | - |
| - Hydroxypropyl chitosan POLYMERE HYDROPHILE | - | 0,12 | - | - |
| - Alcool polyvinylique POLYMERE HYDROPHILE | 0,2 | - | - | - |
| - Copolymères acrylate d'éthyle méthacrylate de méthyle réticulé en dispersion aqueuse à 50% en MA* (Daitosol 5000 AD de Daito) POLYMERE HYDROPHILE | - | - | 4,5 | - |
| Copolymère Acrylamide/AMPS Na à 40% en matières sèches dans isohexadécane avec polysorbate 80 ( Simulgel 600^{®} de SEPPIC) POLYMERE HYDROPHILE | - | - | - | 3,5 |
| - Copolymère Styrène/Acrylates/ammonium methacrylate, à 40% MA dans l'eau avec butylene glycol et sodium laureth-12 sulfate (Syntran 5760) POLYMERE HYDROPHILE | - | - | - | 20 |
| - Polyvinylpyrrolidone POLYMERE HYDROPHILE | - | - | 1 | - |
| - Copolymère éthylène diamine/dilinoléate de stéaryle (Uniclear 100 VG^{®}" d'ARIZONA CHEMICAL | - | - | 5 | - |
| - Copolymère réticulé trimethylpentanediol/ acide adipique/glycérol (Lexorez 200 de INOLEX) | - | - | 1 | - |
| - Copolymère acrylate/methacrylate d'ammanium à 50% en MA dans l'eau avec sodium lauryl sulfate (Ultragel 2075C de GANZ Chem.) POLYMERE HYDROPHILE | - | - | 5,6 | - |
| - Mélange cyclopentasiloxane, polymère réticulé dimethicone/vinyl dimethicone et laureth-4 dans l'eau à 17% en MA (Jeesil LTX de JEEN Int) | - | - | 1,19 | - |
| - Copolymère acrylique (ACP 10 de 3M) dans la phényltriméthicone(commercialisé sous la référence MSX 5381 par 3M) | - | 0,2 | - | - |
| - Butylène glycol | - | - | - | 3,9 |
| - Pentylène glycol | - | - | 2 | - |
| - Poydiméthysiloxane oxypropylénée (20 OP)/oxyethylénée (20OE) (DC Q2-5220 Resin Modifier de Corning) | 0,2 | - | - | - |
| - Acide stéarique | 5,45 | 5,82 | 3,4 | - |
| - PEG-40 stearate (Myrj 52P d'UNICHEMA) | - | 0,5 | - | - |
| - PEG-200 glyceyl stearate | - | - | - | 4 |
| - Triéthanolamine | 2,4 | 2,4 | 1,6 | - |
| - Aminométhyl propanediol | - | 0,5 | - | - |
| - Fibres de polyimide-amide (Kermel Tech, 2Dtex, 2 mm de Kermel) | - | - | 0,1 | - |
| - Fibre de cellulose ("Natural rayon flock fiber RC1BE - N003 - M04 » de Claremont Flock | - | 0,25 | 0,75 | 1 |
| - Oxyde de fer noir | 8 | 7,2 | 6 | 7 |
| - Talc | - | 2 | - | - |
| - Simethicone | 0,12 | 0,13 | 0,10 | 0,10 |
| - BHT | 0,1 | - | - | - |
| - Disodium EDTA | - | - | 0,1 | 0,2 |
| - Sodium dehydrocetate | - | - | - | 0,2 |
| - Panthenol | 1 | 0,45 | - | - |
| - Conservateurs | qs | qs | qs | qs |
| - Alcool | - | - | - | 3 |
| - Eau | Qsp 100 | Qsp 100 | Qsp 100 | Qsp 100 |

| | | | | |
|---|---|---|---|---|
| *MA = matières actives | | | | |

| | | | | |
|---|---|---|---|---|
| **Teneur totale en Cires** | 23,5 | 18,6 | 16,8 | 10,9 |
| **Teneur totale en polymères hydrophiles** | 1,94 | 4,79 | 6,25 | 9,40 |

### Exemple 5 à 7 : Compositions (ii)

| | **Exemple 5** | **Exemple 6** | **Exemple 7** |
|---|---|---|---|
| - Cire de carnauba | 3,2 | 3,4 | 6 |
| - Cire d'abeille | 4,7 | 4,37 | 5,2 |
| - Cire de candelilla | 0,3 | - | 2 |
| - Cire de paraffine | 13, 81 | 13,82 | - |
| - Cire obtenue par hydrogénation d'huile d'olive estérifiée avec l'alcool stéarylique (PHYTOWAX Olive 18 L 57 de SOPHIM) | 0,1 | 0,2 | - |
| - Huile de palme totalement hydrogénée (GV60 de Sio) | - | 0,2 | - |
| - Cire de son de riz | 0,1 | - | 5,6 |
| - (Hydroxystéaryloxy)stéarate d'alkyle en C18-C38 (Kester K82 P de Koster Keunen) | - | - | 4,1 |
| - Hydroxyéthyl cellulose quaternisée par chlorure de 2,3 époxypropyl triméthyl ammonium POLYMERE HYDROPHILE | 0,1 | 0,1 | 0,1 |
| - Hydroxyethylcellulose POLYMERE HYDROPHILE | 0,89 | 0,89 | 0,88 |
| - Polyméthacrylate de sodium dans l'eau à 25 % en MA* (Darvan 7 de Vanderbilt) POLYMERE HYDROPHILE | 1 | 1 | 1 |
| - Gomme arabique POLYMERE HYDROPHILE | 3,39 | 3,39 | 3,38 |
| - Copolymères acrylate d'éthyle méthacrylate de méthyle réticulé en dispersion aqueuse à 50% en MA* (Daitosol 5000 AD de Daito) POLYMERE HYDROPHILE | - | - | 1 |
| - Poydimethysiloxane oxypropylénée (20 OP)/oxyethylénée (200E) (DC Q2-5220 Resin Modifier de Corning) | - | - | 0,2 |
| - Mélange de cyclopentasiloxane et dimethiconol (DC1501 Fluid) | 8 | - | - |
| - Acide stearique | 5,65 | 6,6 | 5,82 |
| - Triethanolamine | 2,4 | 2,4 | 2,4 |
| - Stearate PEG-40 (Myrj 52P d'UNICHEMA) | - | 0,5 | 1,5 |
| - Simethicone | 0,13 | 0,13 | 0,13 |
| - Oxyde de fer noir | 7 | 7,14 | 7,14 |
| - Ultramarines | - | 2,1 | - |
| - Acide palmitique | 0,2 | - | - |
| - Aminomethyl propanediol | 0,39 | 0,8 | 0,5 |
| - BHT | 0,1 | 0,1 | 0,1 |
| - Panthenol | 0,5 | 0,01 | 0,5 |
| - Conservateurs | qs | qs | qs |
| - Eau | Qsp 100 | Qsp 100 | Qsp 100 |

| | | | |
|---|---|---|---|
| **Teneur totale en Cires** | 21,90 | 22,04 | 22,90 |
| **Teneur totale en polymères hydrophiles** | 4,63 | 4,63 | 5,11 |

Toutes les associations de compositions (i) et (ii) telles que décrites ci-dessus peuvent être envisagées.

Selon un mode de réalisation particulier, on applique à titre de composition (i) celle décrite en exemple 2, et à titre de composition (ii) celle décrite en exemple 7. On obtient ainsi, en particulier lorsque la composition (ii) est appliquée sur au plus le tiers extérieur de la frange de cils, un effet maquillage particulier conforme à celui qui est représenté en figure 43, à savoir qui ouvre le regard et agrandit l'oeil, en modifiant optiquement la perception de la forme de l'oeil.

## Revendications

1. Kit de maquillage et/ou de soin des cils **caractérisé en ce qu'**il comprend ;
- au moins une composition (i) présentant une teneur totale en cire(s) et en polymère(s) hydrophile(s) inférieure ou égale à 26 % en poids par rapport au poids total de la composition,
- au moins une composition (ii) présentant une teneur total en cire(s) et en polymère(s) filmogène(s) hydrophile(s) supérieure à 26 % en poids par rapport au poids total de la composition,
au moins l'une desdites compositions (i) et (ii) comprenant une phase aqueuse continue, et **en ce qu'**il comprend un moyen d'application sur la frange de cils, comportant une pluralité d'éléments d'application (6 ; 60) disposés sur un support (5) sous forme d'au moins une rangée dont la longueur est telle que les éléments d'application (6 ; 60) puissent au mieux contacter simultanément au plus un quart des cils de la frange.

2. Kit selon la revendication 1, **caractérisé par** le fait le moyen d'application comporte :
- une tige (4),
- un support (5 ; 61) relié à la tige (4) et s'étendant selon un axe longitudinal (Y),
- une pluralité d'éléments d'application (6 ; 60) disposés sur le support (5) et s'étendant sensiblement transversalement à l'axe longitudinal (Y), les éléments d'application (6 ; 60) s'étendant sur une distance le long de l'axe longitudinal (Y) du support qui est telle que les éléments d'application (6 ; 60) puissent au mieux contacter simultanément au plus un quart des cils de la frange, l'axe longitudinal (Y) étant orienté sensiblement tangentiellement à la frange.

3. Kit selon la revendication 1 ou 2, **caractérisé par le fait que** les éléments d'application présentent des extrémités libres (30) dont la distance à l'axe longitudinal (Y) varie de manière non monotone lorsque l'on se déplace entre les éléments d'application extrêmes de la rangée (10).

4. Kit selon l'une quelconque des revendications 1 à 3, **caractérisé par le fait que** le nombre d'éléments d'application (6) de la rangée (10) est compris entre 3 et 9, de préférence entre 3 et 8.

5. Kit selon l'une quelconque des revendications 1 à 4, **caractérisé par le fait que** les éléments d'application (6) comportent des dents.

6. Kit selon l'une quelconque des revendications 1 à 4, **caractérisé par le fait que** les éléments d'application (6) comportent des poils.

7. Kit selon l'une quelconque des revendications précédentes, **caractérisé par le fait que** les éléments d'application (6) s'étendent sur le support (5) sur une distance (*d*) comprise entre 0,1 et 1 cm environ, notamment inférieure ou égale à 8 mm, voire 7 mm ou 6 mm, notamment comprise entre 2 et 5 mm environ, notamment entre 2 et 3 mm environ.

8. Kit selon l'une quelconque des revendications précédentes, **caractérisé par le fait que** les éléments d'application (6) sont disposés selon au moins deux rangées (10).

9. Kit selon la revendication précédente, **caractérisé par le fait que** les éléments d'application (6) d'une première rangée et ceux d'une deuxième rangée s'étendent dans des directions différentes.

10. Kit selon la revendication 8, **caractérisé par le fait que** les éléments d'application (6) d'au moins une première rangée et ceux d'une deuxième rangée s'étendent dans des directions parallèles.

11. Kit selon la revendication 8, **caractérisé par le fait que** les rangées (10) sont disposées à l'opposé l'une de l'autre relativement au support (5).

12. Kit selon l'une quelconque des revendications précédentes, **caractérisé par le fait que** la hauteur des éléments d'application (6) est comprise entre 0,5 et 10 mm environ, notamment entre 1 et 3 mm environ.

13. Kit selon la revendication 1 ou 2, **caractérisé par le fait qu'**il comporte un peigne.

14. Kit selon la revendication 1 ou 2, **caractérisé par le fait qu'**il comporte une brosse.

15. Kit selon l'une quelconque des revendications précédentes, **caractérisé en ce que** les deux compositions (i) et (ii) comprennent une phase continue aqueuse.

16. Kit selon l'une quelconque des revendications précédentes, **caractérisé en ce que** la composition (i) présente une teneur totale en cire(s) et en polymère(s) hydrophile(s) inférieure ou égale à 24 % en poids par rapport au poids total de la composition, de préférence inférieure ou égale à 23,5 % en poids.

17. Kit selon l'une quelconque des revendications précédentes, **caractérisé en ce que** la composition (ii) présente une teneur totale en cire(s) et en polymère(s) hydrophile(s) supérieure ou égale à 27 % en poids par rapport au poids total de la composition, de préférence supérieure ou égale à 28 % en poids.

18. Kit selon l'une quelconque des revendications 1 à 17, **caractérisé en ce que** la composition (i) présente une teneur totale en cire(s) et en polymère(s) hydrophile(s) supérieure ou égale à 10 % en poids par rapport au poids total de la composition, de préférence supérieure ou égale à 15 % en poids.

19. Kit selon l'une quelconque des revendications 1 à 18, **caractérisé en ce que** la composition (ii) présente une teneur totale en cire(s) et en polymère(s) hydrophile(s) allant jusqu'à 50 % en poids par rapport au poids total de la composition.

20. Kit selon l'une quelconque des revendications 1 à 19, **caractérisé en ce que** la cire est présente dans chacune des compositions (i) et (ii) en une teneur allant de 0,1 à 50% en poids, mieux de 1 à 40 % en poids et encore mieux allant de 5 à 30 % en poids, par rapport au poids total de la composition.

21. Kit selon l'une quelconque des revendications précédentes, **caractérisé en ce que** la teneur en cire(s) dans la composition (i) varie de 1 à 40 %, de préférence de 5 à 30 %, et mieux de 8 à 25 % en poids, par rapport au poids total de la composition.

22. Kit selon l'une quelconque des revendications précédentes, **caractérisé en ce que** la teneur en cire(s) dans la composition (ii) varie de 5 à 40 %, de préférence de 15 à 30 %, et mieux de 18 à 25 % en poids, par rapport au poids total de la composition.

23. Kit selon l'une quelconque des revendications précédentes, **caractérisé en ce que** la teneur totale dans la composition (i) en matières sèches de polymère(s) hydrophile(s) est comprise entre 0,5 et 15 %, de préférence entre 1 et 10 % en poids, par rapport au poids total de la composition.

24. Kit selon l'une quelconque des revendications précédentes, **caractérisé en ce que** la teneur totale dans la composition (ii) en matières sèches de polymère(s) hydrophile(s) est comprise entre 1 et 10 % en poids, par rapport au poids total de la composition.

25. Kit selon l'une quelconque des revendications précédentes, **caractérisé en ce que** chacune des composition (i) ou (ii) comprend en outre au moins un polymère additionnel lipophile qui est soluble dans une phase solvant non aqueuse ou en dispersion dans une phase solvant non aqueuse.

26. Kit selon l'une quelconque des revendications précédentes, **caractérisé en ce que** la composition (i) ou (ii) contient un terpolymère résultant de la copolymérisation :
- d'au moins un monomère A choisi parmi les esters issus de la réaction de l'acide (meth)acrylique avec un monoalcool comprenant de 2 à 20 atomes de carbone,
- d'au moins un monomère B choisi parmi les esters issus de la réaction de l'acide méthacrylique avec un monoalcool comprenant de 1 à 10 atomes de carbone,
- d'au moins un monomère C choisi parmi les N-vinyl lactames.

27. Kit selon l'une quelconque des revendications précédentes, **caractérisé en ce que** la différence entre la teneur totale en cire(s) et en polymère(s) hydrophile(s) de la composition (ii) et la teneur totale en cire(s) et en polymère(s) hydrophile(s) de la composition (i) est supérieure ou égale à 2 %, voire à 3 %, et mieux à 4 % en valeur absolue.

28. Kit selon l'une quelconque des revendications précédentes, **caractérisé en ce que** :
- la composition (i) comprend une phase aqueuse continue, au moins une cire dite collante, possédant un collant supérieur ou égal à 0,7 N.s et une dureté inférieure ou égale à 3,5 MPa, au moins l'association d'un polymère hydrophile cationique et d'un polymère hydrophile anionique et au moins un terpolymère acrylique tel que décrit selon la revendication 26, et
- la composition (ii) comprend une phase aqueuse continue, au moins une cire dite collante, possédant un collant supérieur ou égal à 0,7 N.s et une dureté inférieure ou égale à 3,5 MPa, au moins l'association d'un polymère hydrophile cationique et d'un polymère hydrophile anionique et au moins un polymère filmogène en dispersion dans une phase aqueuse.

29. Kit selon l'une quelconque des revendications précédentes, **caractérisé en ce que** les compositions (i) et (ii) comprennent en outre au moins une huile et/ou un système émulsionnant et/ou une matière colorante et/ou une charge et/ou des fibres et/ou des actifs cosmétiques.

30. Kit selon l'une quelconque des revendications précédentes, **caractérisé en ce qu'**il se présente dans un seul et même conditionnement, notamment sous forme d'un récipient délimitant au moins un compartiment qui comprend la composition (i) et au moins un compartiment qui comprend la composition (ii), lesdits compartiments étant éventuellement fermés par un élément de fermeture.

31. Procédé de maquillage et/ou de soin non thérapeutique des cils, **caractérisé en ce qu'**il comprend :
- au moins une étape d'application sur les cils d'au moins une couche d'une composition (i) présentant une teneur total en cire(s) et en polymère(s) hydrophile(s) inférieure ou égale à 26 % en poids par rapport au poids total de la composition, et
- au moins un étape d'application d'au moins une couche d'une composition (ii), présentant une teneur totale en cire(s) et en polymère(s) filmogène(s) hydrophile(s) supérieure à 26 % en poids par rapport au poids total de la composition, au moins l'une desdites compositions (i) et (ii) comprenant une phase aqueuse continue, et l'étape d'application de la composition (ii) étant effectuée à l'aide du moyen d'application tel que défini selon l'une quelconque des revendications 1 à 14.

32. Procédé selon la revendication 31, **caractérisé en ce que** la composition (i) est appliquée préalablement à la composition (ii).

33. Procédé selon la revendication 32, **caractérisé en ce que** la composition (ii) est appliquée sur seulement une portion de la frange des cils.

34. Procédé selon l'une quelconque des revendications 31 à 33, **caractérisé en ce que** la composition (i) est appliquée sur l'ensemble de la frange des cils et la composition (ii) est appliquée seulement sur au plus un tiers de la frange des cils, de préférence sur le tiers extérieur de la frange des cils, voire sur au plus un quart de ladite frange.

35. Support maquillé comprenant un maquillage susceptible d'être obtenu selon le procédé tel que défini selon l'une quelconque des revendications 31 à 34.
